# EUROPEAN PATENT SPECIFICATION

(11) **EP 2 057 930 B1**
(45) Date of publication and mention of the grant of the patent: **04.04.2012**
(21) Application number: 07792486.8
(22) Date of filing: 14.08.2007
(51) Int. Cl.: A61B 1/04, G02B 23/24

(54) **DISTAL END HOOD FOR ENDOSCOPE AND ENDOSCOPE WITH HOOD**
DISTALE ENDHAUBE FÜR ENDOSKOP UND ENDOSKOP MIT HAUBE
CAPUCHON D'EXTREMITE DISTAL POUR ENDOSCOPE ET ENDOSCOPE AVEC CAPUCHON

(30) Priority: 30.08.2006 JP 2006234083
(43) Date of publication of application: 13.05.2009
(73) Proprietor: Olympus Medical Systems Corp., Tokyo 151-0072 (JP)
(72) Inventor: ICHIMURA, Hironobu, Tokyo 192-8512 (JP)
(74) Representative: von Hellfeld, Axel
(86) International application number: PCT/JP2007/065843
(87) International publication number: WO 2008/026445

(56) References cited:
- JP-A- 11 342 104
- JP-A- 2003 038 415
- JP-A- 2003 245 244
- JP-A- 2004 129 951
- JP-A- 2005 000 640
- JP-A- 2007 151 873
- US-A1- 2004 158 129
- US-A1- 2004 267 092
- US-A1- 2005 052 753

## Description

### Technical Field

The present invention relates to a distal end hood for an endoscope provided with an observation optical system of an object contact type which brings a distal end portion of an objective optical system in contact with an object to observe the object, and an endoscope with a distal end hood.

### Background Art

Jpn. Pat. Appln. KOKAI Publication No. 55-84141 (Patent document 1) describes an endoscope with a hood provided at a distal end portion of an insertion portion of the endoscope. Here, such a configuration is adopted that a distance between a distal end face of the insertion portion and a surface of a body tissue is kept constant by pressing a distal end portion of the hood on the body tissue. In the endoscope described in the Patent document 1, the hood is made of an elastic body. Thereby, when the distal end portion of the hood is pressed to the body tissue, the former can be brought in soft close contact with the latter, so that the body tissue is prevented from being injured.

Jpn. Pat. Appln. KOKAI Publication No. 2005-640 (Patent document 2) describes an endoscope provided with an observation optical system of an object contact type and an ordinary observation optical system. The observation optical system of an object contact type brings a distal end portion of an objective optical system in contact with an object to observe the object. The abovementioned ordinary observation optical system observes an object in a non-contact state of the objective optical system with the object. In the endoscope described in the Patent document 2, a projecting portion projecting forward is provided on a distal end face of an insertion portion of the endoscope, and the observation optical system of an object contact type is disposed on an end face of the projecting portion. Further, an observation window for the ordinary observation optical system, illumination windows for a plurality of illumination optical systems, a gas-feeding and water-feeding nozzle, a distal end opening of a treatment tool insertion channel, and the like are disposed on an end face on a proximal end side of the projecting portion of the insertion portion.

### Disclosure of invention

Document US 2004/267092 A1 discloses a distal end hood for an endoscope, including a cylindrical hood main body attached to an endoscope at a distal end portion of an insertion portion inserted into a lumen, a proximal end portion of the hood main body being attached to an outer peripheral surface of the distal end portion, wherein an elastically deformable elastic deformation portion is disposed at a distal end portion of the hood main body, wherein the elastic deformation portion is configured such that a distal end portion of the elastic deformation portion abuts on the analyte for observation beyond near-field. The elastic deformation portion is elastically deformable for near-field observation.

Document US 2004/158129 A1 discloses an endoscope having several observation portions which are arranged above each other and which have different distances to an analyte.

In the endoscope described in the Patent document 1, such a configuration that a hood made of an elastic body is provided at the distal end portion of the endoscope is shown. The hood made of the elastic body described in the Patent document 1 has such a configuration that a distance between the distal end face of the insertion portion and a surface of a body tissue is kept constant by pressing the distal end portion of the hood on the body tissue. However, as described in Patent document 2, when the hood described in the Patent document 1 is assembled to an endoscope provided with an observation optical system of an object contact type which brings a distal end portion of an objective optical system in contact with an object to observe the object, it becomes difficult to bring the distal end portion of the objective optical system in contact with an object due to interference of the hood. Incidentally, since the hood made from an elastic body described in the Patent document 1 has such softness that a body tissue is not injured due to soft close contact of the distal end portion of the hood with the body tissue when the distal end portion is pressed on to the body tissue, it is difficult to elastically deform the distal end portion of the objective optical system up to a position where the distal end portion is brought into contact with the object.

Since a hood is not shown in the endoscope described in the Patent document 2, it is difficult to maintain a constant distance between a position of an observation lens of the ordinary observation optical system and a surface of a body tissue during observation using the ordinary observation optical system. Therefore, it is difficult to perform magnification observation of the surface of the body tissue in a state that the distance between the position of the observation lens of the ordinary observation optical system and the surface of the body tissue is kept constant. Thereby, such a problem arises that it is difficult to perform magnification observation of the surface of the body tissue stably utilizing the ordinary observation optical system.

In view of these circumstances, the present invention has been made and an object thereof is to provide a distal end hood for an endoscope where observation of a surface of a body tissue utilizing an ordinary observation optical system can be performed stably even in an endoscope provided with an observation optical system of an object contact type where observation is performed in contact with a body tissue and object contact observation where observation is performed in a state where the observation optical system of an object contact type has been brought into contact with a body tissue can be performed stably, and also provide an endoscope with a hood.

According to an aspect of the present invention as defined in claim 1, an endoscope with a hood is provided comprising, an insertion portion inserted into lumen, a projecting insertion portion inserted into an lumen, a projecting face provided at a distal end portion of the insertion portion so as to project, a first observation portion disposed on the projecting face and performing observation in a contact state of an observation optical system with an analyte, a second observation portion disposed rearward beyond the projecting face, and performing observation in a non-contact state of the observation optical system with the analyte and allowing switching between ordinary observation state for wide-angle observation and a magnification observation state for magnification observation, and a distal end hood including a cylindrical hood main body whose proximal end portion is attached on an outer peripheral surface of the distal end portion and an elastic deformation portion provided at a distal end portion of the hood main body so as to extend forward beyond the projecting face and which is elastically deformable, wherein the elastic deformation portion is configured such that a distal end portion of the elastic deformation portion abuts on the analyte to position an observation position of the second observation portion at the magnification observation time utilizing the second observation portion, while the elastic deformation portion is elastically deformable up to a position where the first observation portion abuts on the analyte at a contact observation time utilizing the first observation portion.

With this configuration, the observation position of the second observation portion is positioned by causing the distal end portion of the elastic deformation portion of the hood main body to abut on an analyte at a magnification observation time utilizing the second observation portion where an analyte is observed in a non-contact state of the observation optical system with the analyte. The elastic deformation portion is elastically deformed up to a position at which the first observation portion abuts on an analyte at a contact observation time utilizing the first observation portion.

Preferably, the hood main body has such a configuration in which a proximal end portion attached on an outer peripheral surface of the distal end portion is formed integrally with a distal end cover covering the outer peripheral surface of the distal end portion.

With this configuration, by forming the hood main body integrally with the distal end cover covering an outer peripheral surface of the distal end portion, the number of assembling steps can be reduced as compared with the number of steps required to assemble a distal end hood prepared as a separate part to the distal end cover of the insertion portion, which can result in reduction of manufacturing cost.

Preferably, the hood main body is configured such that the distal end portion of the elastic deformation portion is disposed in an observation depth range between a field of view on a far point side of the second observation portion and a field of view on a near point side at the magnification observation time utilizing the second observation portion.

With this configuration, when magnification observation is performed in a state that the distal end portion of the elastic deformation portion has been caused to abut on an analyte to position an observation position of the second observation portion, the distal end portion of the elastic deformation portion is disposed in an observation depth range between the depth of field on the far point side of the second observation portion and the depth of field on the near point side thereof.

Preferably, the hood main body has such a configuration in which a mounting portion mounted on the outer peripheral surface of the distal end portion and the elastic deformation portion are formed as separate parts, respectively.

With this configuration, the mounting portion of the hood main body and the elastic deformation portion can be made of different materials, respectively.

Preferably, the elastic deformation portion is made from a material softer than that for the mounting portion.

With this configuration, by making the elastic deformation portion from material softer than that for the mounting portion, the elastic deformation portion can be elastically deformed easily up to a position at which the first observation portion abuts on an analyte at a contact observation time using the first observation portion.

Preferably, the hood main body has such a configuration in which a mounting portion mounted on the outer peripheral surface of the distal end portion and the elastic deformation portion are made from the same material, and the elastic deformation portion is made of a thin portion smaller in thickness of a wall portion than the mounting portion.

With this configuration, by making the elastic deformation portion from a thin portion smaller in thickness of a wall portion than the mounting portion, the mounting portion of the hood main body and the elastic deformation portion can be made from the same material.

Preferably, the hood main body has such a configuration that a mounting portion mounted on the outer peripheral surface of the distal end portion and the elastic deformation portion are made from the same material, and a wall portion of the elastic deformation portion is made softer than that of the mounting portion by forming a notch portion in a distal end portion of a wall portion of the hood main body.

With this configuration not forming part of the invention, by forming a notch at the distal end portion of the wall portion of the hood main body to make the distal end portion softer than the mounting portion, thereby forming the elastic deformation portion, the mounting portion of the hood main body and the elastic deformation portion can be made from the same material.

Preferably, the hood main body has such a configuration that a mounting portion mounted on the outer peripheral surface of the distal end portion and the elastic deformation portion are made from the same material, and a wall portion of the elastic deformation portion is made softer than that of the mounting portion by forming an opening in a wall portion of the hood main body.

With this configuration not forming part of the invention, by forming an opening in the wall portion of the hood main body to make the wall portion softer than the mounting portion, thereby forming the elastic deformation portion, the mounting portion of the hood main body and the elastic deformation portion can be made from the same material.

Preferably, the elastic deformation portion is configured such that a position of a proximal end portion thereof is disposed rearward beyond a distal end position of the first observation portion.

With this configuration, by arranging a position of the proximal end portion of the elastic deformation portion behind a distal end position of the first observation portion, the elastic deformation portion is elastically deformed reliably up to a position where the first observation portion abuts on an analyte at a contact observation time using the first observation portion.

Preferably, the elastic deformation portion is configured such that a position of a rear end portion of the opening is disposed rearward beyond a distal end position of the first observation portion.

With this configuration not forming part of the invention, by arranging a position of the rear end portion of the opening of the elastic deformation portion behind the distal end position of the first observation portion, the elastic deformation portion is elastically deformed reliably up to a position at which the first observation portion abuts on an analyte at a contact observation time using the first observation portion.

Preferably, the elastic deformation portion is configured such that a position of a proximal end portion of the thin portion is disposed rearward beyond a distal end position of the first observation portion.

With the configuration not forming part of the invention, by arranging a position of the proximal end portion of the thin portion of the elastic deformation portion behind the distal end portion of the first observation portion, the elastic deformation portion is elastically deformed reliably up to a position at which the first observation portion abuts on an analyte at a contact observation time using the first observation portion.

Preferably, the elastic deformation portion is configured such that a position of a proximal end portion of the notch portion is disposed rearward beyond a distal end position of the first observation portion.

With the configuration not forming part of the invention, by arranging the position of the proximal end portion of the notch portion of the elastic deformation portion behind the distal end position of the first observation portion, the elastic deformation portion is elastically deformed reliably up to a position at which the first observation portion abuts on an analyte at a contact observation time using the first observation portion.

According to another aspect of the present invention as defined in claim 8, an endoscope with a hood is provided comprising, an insertion portion inserted into a lumen, a zoom optical system which is disposed at a distal end portion of the insertion portion and can be switched between a contact observation state in which observation is performed in a contact state of an observation optical system with an analyte and a non-contact observation state in which observation is performed in a non-contact state of the observation optical system with the analyte, wherein the observation optical system can be switched between an ordinary observation state for wide-angle observation and a magnification observation state for magnification observation at the non-contact observation state time, and a distal end hood including a cylindrical hood main body whose proximal end portion is attached on an outer peripheral surface of the distal end portion and an elastic deformation portion provided at a distal end portion of the hood main body so as to extend forward beyond the projecting face and which is elastically deformable, wherein the elastic deformation portion is configured such that a distal end portion of the elastic deformation portion abuts on the analyte to position an observation position at the magnification observation time of the zoom optical system at the magnification observation time, while the elastic deformation portion is elastically deformable up to a position where the zoom optical system abuts on the analyte at the contact observation time of the zoom optical system.

With this configuration, by causing the distal end portion of the elastic deformation portion of the hood main body to abut on an analyte, an observation position of the zooming optical system is positioned at a magnification observation time in a non-contact observation state where the analyte is observed in a non-contact state of the observation optical system with the analyte. The elastic deformation portion is elastically deformed up to a position at which the zooming optical system abuts on an analyte at a contact observation time in the contact observation state.

According to the present invention, a distal end hood for an endoscope and an endoscope with a hood can be provided where observation of a surface of a body tissue utilizing an ordinary observation optical system can be performed stably even in an endoscope provided with an observation optical system of an object contact type where observation is performed in a contact state with an analyte and object contact observation where observation is performed in a contact state of an observation optical system of an object contact type with a body tissue can be performed stably.

Brief Description of Drawings
FIG. 1 is a schematic configuration diagram of an entire system of an endoscope according to a first embodiment of the present invention;
FIG. 2 is a vertical sectional view showing an internal structure of a distal end portion of the endoscope according to the first embodiment;
FIG. 3 is a front view of the distal end portion of the endoscope according to the first embodiment;
FIG. 4 is a vertical sectional view showing an observation optical system for ordinary observation assembled in the distal end portion of the endoscope according to the first embodiment;
FIG. 5 is a vertical sectional view of a main portion showing a cell observation state utilizing an observation optical system of an object contact type of the endoscope according to the first embodiment;
FIG. 6A is a vertical sectional view of a main portion showing an ordinary observation state of the endoscope according to the first embodiment;
FIG. 6B is a vertical sectional view of a main portion showing a zoom observation state;
FIG. 6C is a vertical sectional view of a main portion showing a contact observation state;
FIG. 7 is a perspective view showing a distal end hood attached on a distal end portion of an endoscope according to a second embodiment;
FIG. 8 is a vertical sectional view of a main portion showing a zoom observation state of an observation optical system for ordinary observation of the endoscope according to the second embodiment;
FIG. 9 is a vertical sectional view of a main portion showing a contact observation state of an observation optical system for contact observation of the endoscope according to the second embodiment;
FIG. 10 is a front view of a distal end portion of the endoscope showing a relationship between a U-shaped groove portion of a modified embodiment of a distal end hood of the endoscope according to the second embodiment and a view range at an ordinary observation time using a second imaging unit for ordinary observation;
FIG. 11 is a sectional view of a distal end portion of the endoscope showing a relationship between the U-shaped groove portion of the modified embodiment of the distal end hood shown in FIG. 10 and a view range of the second imaging unit;
FIG. 12 is a perspective view showing a distal end hood attached to a distal end portion of an endoscope according to a third embodiment of the present invention;
FIG. 13 is a vertical sectional view showing a distal end portion of an endoscope with a hood according to a fourth embodiment of the present invention;
FIG. 14 is a vertical sectional view showing a distal end portion of an endoscope with a hood according to a fifth embodiment of the present invention; and
FIG. 15 is a vertical sectional view of a main portion showing an internal structure of a distal end portion of an endoscope of a single-lens type according to a sixth embodiment of the present invention.

### Best Mode for Carrying Out the Invention

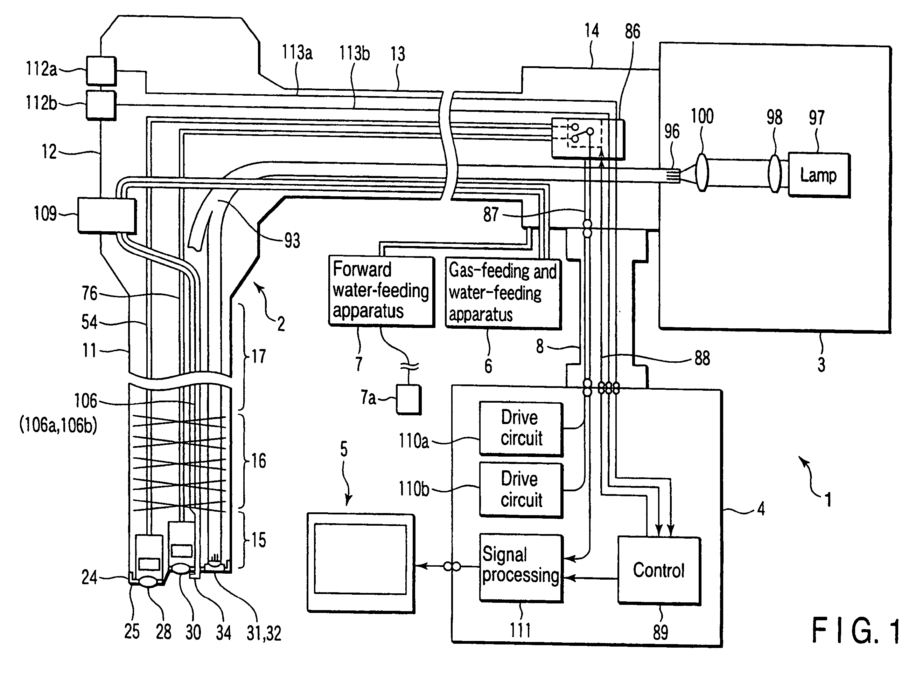

A first embodiment of the present invention will be explained below with reference to FIG. 1 to FIG. 6C. FIG. 1 shows a schematic configuration of the entire endoscope system 1 according to the embodiment. As shown in FIG. 1, the endoscope system 1 according to the embodiment is provided with an endoscope 2, a light source apparatus 3 serving as illumination means supplying illumination light to the endoscope 2, a processor 4 serving as a signal processing unit performing signal processing to the endoscope 2, a monitor 5 connected to the processor 4, and a gas-feeding and water-feeding apparatus 6 performing gas-feeding and water-feeding, and a forward water-feeding apparatus 7 performing forward water-feeding.

The endoscope 2 includes an elongated insertion portion 11 inserted into a body cavity, an operation portion 12 coupled to a proximal end of the insertion portion 11, and a universal cable 13 extending from a side portion of the operation portion 12. A connector 14 provided at an end portion of the universal cable 13 is connected to the light source apparatus 3 in an attachable and detachable manner. Further, the connector 14 is connected to the processor 4 via a scope cable 8.

The insertion portion 11 of the endoscope 2 includes a hard distal end portion 15 formed at a distal end portion thereof, a bending portion 16 formed at a proximal end of the distal end portion 15, and a flexible tubular portion 17 with flexibility formed so as to extend from a proximal end of the bending portion 16 to the operation portion 12.

The bending portion 16 is continuously provided with a plurality of annular bending pieces extending in an axial direction of the insertion portion 11 in a rotational movable manner. A bending blade formed by knitting thin wires in a cylindrical shape is fitted on the plurality of bending pieces so as to cover outer peripheries thereof. An outer skin 21 is fitted on the bending blade so as to maintain water-tightness. The outer skin 21 performs covering such that the insertion portion 11 comprising the distal end portion 15, the bending portion 16, and the flexible tubular portion 17 are integrated over its whole length.

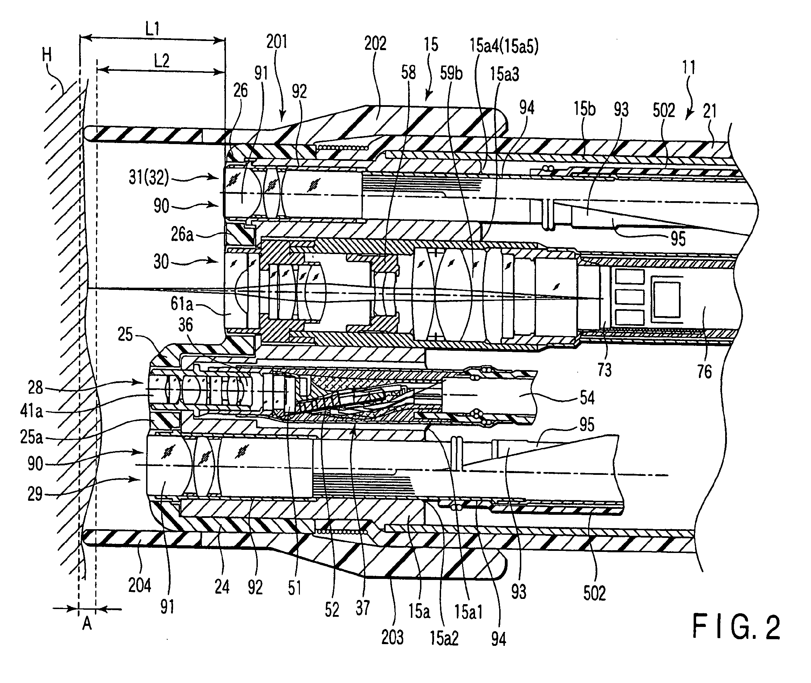

FIG. 2 shows an internal configuration of the distal end portion of the insertion portion 11 of the endoscope 2 according to the embodiment. A cylindrical member (a distal end hard member) 15a made of a hard metal, and an annular reinforcing ring 15b fitted on an outer peripheral portion of the cylindrical member 15a positioned on a proximal end side thereof are disposed within the distal end portion 15 of the insertion portion 11. A plurality of (eight (first to eighth) in this embodiment) hole portions 15a1 to 15a8 parallel to the axial direction of the insertion portion 11 are formed in the cylindrical member 15a. A proximal end portion of the reinforcing ring 15b is coupled to the foremost end bending piece of the bending portion 16.

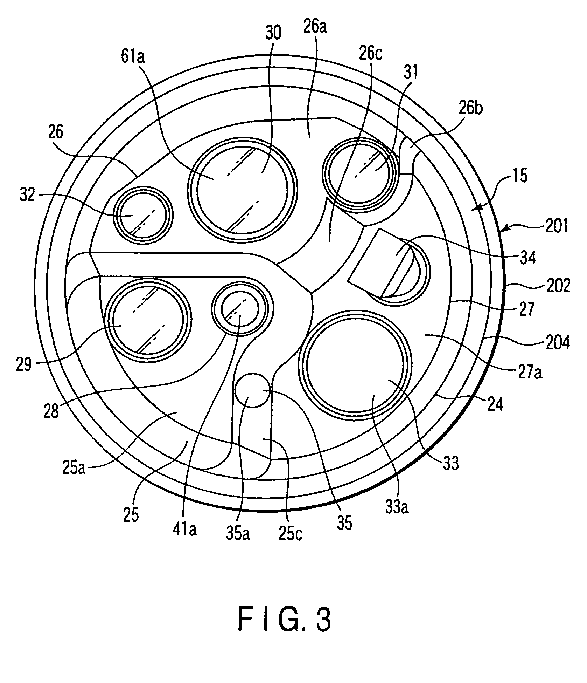

Further, a distal end cover 24 disposed at the distal end portion 15 of the insertion portion 11 is attached to a distal end face of the cylindrical member 15a and a distal end outer peripheral portion of the cylindrical member 15a so as be fitted thereon. As shown in FIG. 3, the distal end cover 24 is formed with three step portions 25, 26, and 27; a projecting step portion 25 projecting forward, a middle step portion 26 lower than the projecting step portion 25 by one step, and a lower step portion 27 lower than the middle step portion 26 by one step. Here, an end face of the projecting step portion (projecting portion) 25 is formed by a flat surface 25a perpendicular to the axial direction of the insertion portion 11. A projecting face is formed by the flat surface 25a of the projecting step portion 25.

As shown in FIG. 3, the flat surface 25a of the projecting step portion 25 is formed so as to occupy an area of about 1/4 of a whole circular front surface of the distal end cover 24 in the embodiment. That is, the flat surface 25a is formed so as be positioned in a region defined by a lower half of the whole circular front surface of the distal end cover 24 and a left side portion thereof regarding a center line extending vertically.

A first lens 41a which is an observation lens of a first imaging unit (first observation portion) 28 of an object contact type, described later, and a first illumination window (first illumination portion) 29 are disposed on the flat surface 25a of the projecting step portion 25. The first imaging unit 28 is positioned at an approximately central portion of the distal end portion 15 of the first imaging unit 28. The first illumination window 29 is disposed at a position near the first imaging unit 28.

The middle set portion 26 includes a flat surface 26a approximately parallel to the flat surface 25a of the projecting step portion 25. A first lens 61a which is an observation lens of a second imaging unit (second observation portion) 30 for ordinary, observation described later, and two (second and third) illumination windows (illumination portions) 31 and 32 are disposed on the flat surface 26a of the middle step portion 26. The second and third illumination windows 31 and 32 are disposed on both sides of the second imaging unit 30.

The lower step portion 27 includes a flat surface 27a approximately parallel to the flat surface 25a of the projecting step portion 25. A distal end opening 33a of a treatment tool insertion channel (also called "forceps channel") 33 disposed within the insertion portion 11 and a gas-feeding and water-feeding nozzle 34, described later, are disposed on the flat surface 27a of the lower step portion 27.

Further, an inclination surface 26b with an inclination angle of, for example, about 45° and a fluid guide face 26c with an inclination angle smaller than the inclination surface 26b are formed on a wall portion between the lower step portion 27 and the middle step portion 26. The fluid guide face 26c is disposed between the gas-feeding and water-feeding nozzle 34 of the lower step portion 27 and the second imaging unit 30 of the middle step portion 26. The fluid guide face 26c is formed by a gentle inclination surface with an angle of about 18°, for example.

The gas-feeding and water-feeding nozzle 34 is a tubular member bent in an approximately L-shaped manner. A distal end portion of the gas-feeding and water-feeding nozzle 34 is disposed toward the first lens 61a which is the observation lens of the second imaging unit 30. Further, a jetting port 34a at a distal end opening of the gas-feeding and water-feeding nozzle 34 is disposed so as to face the fluid guide face 26c. A distal end face of the jetting port 34a at the distal end opening of the gas-feeding and water-feeding nozzle 34 and the first lens 61a, which is the observation lens of the second imaging unit 30, are disposed so as to be flush with each other. Thereby, draining easiness at a cleaning time can be improved.

Incidentally, as described later, the gas-feeding and water-feeding nozzle 34 is connected to a gas-feeding and water-feeding pipeline 106, junction to one flow occurring on its distal end side, and a proximal end side of the gas-feeding and water-feeding pipeline 106 branches into a gas-feeding pipeline 106a and a water-feeding pipeline 106b.

A non-projecting face is formed by portions of the front surface of the distal end cover 24 other than the projecting face which is the flat surface 25a of the projecting step portion 25, for example, the flat surface 26a of the middle step portion 26, the flat surface 27a of the lower step portion 27, an inclination surface 25b of the wall portion between the middle step portion 26 and the projecting step portion 25, an inclination surface 26b of the wall portion and the fluid guide face 26c between the lower step portion 27 and the middle step portion 26, and an inclination surface 25c of the wall portion between the lower step portion 27 and the projecting step portion 25. The inclination surface 25c is formed to have an inclination angle of about 45°, for example.

Further, in the distal end portion 15 of the insertion portion 11, a forward water-feeding opening 35a is disposed on the non-projecting face, i.e., the inclination surface 25c between the lower step portion 27 and the projecting step portion 25 in the embodiment. As shown in FIG. 3, the forward water-feeding opening 35a is disposed near a vertical center axis of the second imaging unit 30 for ordinary observation. The opening 35a communicates with the forward water-feeding pipeline (forward water-feeding channel) 35 inserted into the insertion portion 11.

The eight (first to eighth) hole portions 15a1 to 15a8 in the cylindrical member 15a of the distal end portion 15 are provided at positions corresponding to the first imaging unit 28, the first illumination window 29, the second imaging unit 30, the second illumination window 31, the third illumination window 32, the distal end opening 33a of the treatment tool insertion channel 33, the gas-feeding and water-feeding nozzle 34, and the forward water-feeding opening 35a of the distal end cover 24, respectively. Constituent elements for the first imaging unit 28, constituent elements for the first illumination window 29, constituent elements for the second imaging unit 30, constituent elements for the second illumination window 31, constituent elements for the third illumination window 32, constituent elements for the pipeline of the treatment tool insertion channel 33, constituent elements for the pipeline of the gas-feeding and water-feeding nozzle 34, and constituent elements for the pipeline communicating with the forward water-feeding opening 35a are assembled in the first hole portion 15a1, the second hole portion 15a2, the third hole portion 15a3, the fourth hole portion 15a4, the fifth hole portion 15a5, the sixth hole portion 15a6, the seventh hole portion 15a7, and the eighth hole portion 15a8 in a manner described below, respectively.

The first imaging unit 28 includes a first lens unit 36 of super high power and a first electric part unit 37. Incidentally, the "super high power of the first lens unit 36" is a magnification falling within a level of histological observation involving cells or ducts (for example, a magnification of about 200 to 1000, approximately equal to a magnification of an ordinary optical microscope).

The first electric part unit 37 is continuously connected to a rear end portion of the first lens unit 36. Here, the first electric part unit 37 includes a first imaging element 51 such as a CCD (Charge Coupled Device) or a CMOS (Complementary Metal-Oxide Semiconductor) and a first circuit board 52. Thereby, the first lens unit 36 and the first electric part unit 37 are integrated so that an observation optical unit of super high power is formed.

An optical image focused on the first imaging element 51 from the first lens unit 36 is photoelectrically converted to electrical image signals by the first imaging element 51 and the image signals are output to the first circuit board 52. Further, the electrical signals of the optical image output from the first circuit board 52 are transmitted to a subsequent electrical device, described later, via a signal cable 54.

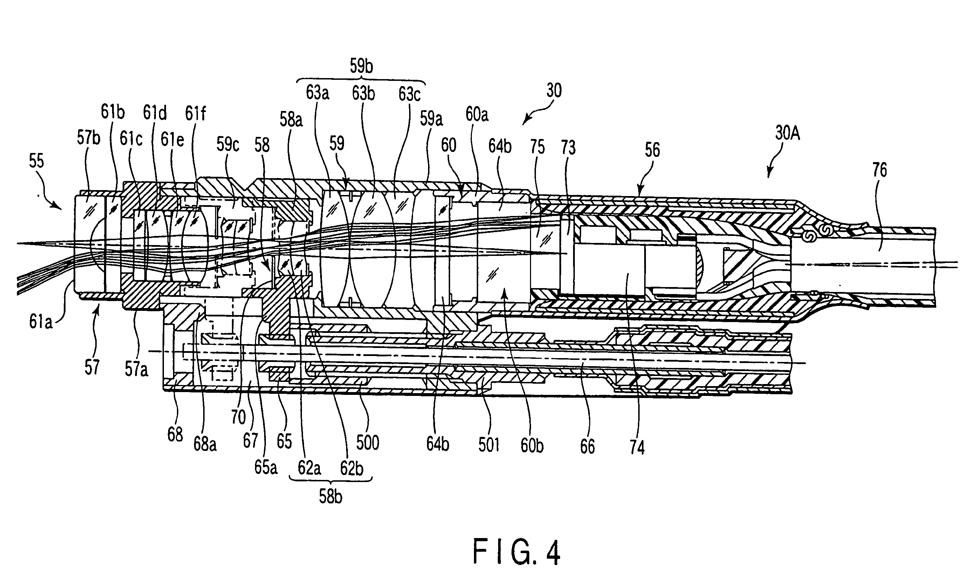

FIG. 4 shows a configuration of the second imaging unit 30 for ordinary observation. That is, the second imaging unit 30 includes a second lens unit 55 provided with a zoom optical system (for example, a magnification level of about 20 to 100) whose observation magnification can be continuously changed from Tele (magnification) position to Wide (wide-angle) position, and a second electric part unit 56.

The second lens unit 55 further includes four (first to fourth) unit configuration bodies 57 to 60. The first unit configuration body 57 includes a first lens frame 57a and a first lens group 57b. As shown in FIG. 4, the first lens group 57b includes six (first to sixth) objective lenses 61a to 61f. Here, the first lens 61a, which is an observation lens, is disposed at a distal end portion of the first lens frame 57a. The first lens 61a is bonded and fixed to, for example, the first lens frame 57a in a state that a distal end portion thereof projects beyond a distal end portion of the first lens frame 57a.

The second constitution body 58 is a moving optical unit for zooming which can advance and retreat in an imaging optical axis direction. The second unit constitution body 58 includes a second lens frame (sliding lens frame) 58a and a second lens group (zoom lens) 58b. The second lens group 58b includes two (first and second) lenses 62a and 62b.

The third unit configuration body 59 includes a third lens frame 59a and a third lens group 59b. A guide space 59c for holding the second unit configuration body 58 so as to advance and retreat in the imaging optical axis direction is provided within the third lens frame 59a on a distal end side thereof. The third lens group 59b is disposed behind the guide space 59c. The third lens group 59b includes three (first to third) lenses 63a to 63c.

The fourth unit configuration body 60 includes a fourth lens frame 60a and a fourth lens group 60b. The fourth lens group 60b includes two (first and second) lenses 64a and 64b.

A projecting portion 65 projecting laterally is provided on one side portion of the second lens frame 58a of the second unit configuration body 58. A distal end portion of an operation wire 66 for operating the second unit configuration body 58 in the imaging optical axis direction in an advancing and retreating manner is fixed to the projecting portion 65.

An operation lever for zooming (not shown) for operating the second unit configuration body 58 which is a zoom optical system between the Wide (wide-angle) position and the Tele (magnification) position in a moving manner is provided on the operation portion 12.

The operation lever for zooming (not shown) provided on the operation portion 12 is operated by a user so that the operation wire 66 is driven in the imaging optical axis direction so as to advance and retreat. At this time, the second unit configuration body 58, which is the zoom optical system, is moved forward (in Wide (wide-angle) position direction) according to operation where the operation wire 66 is pushed out in a direction of the distal end thereof. Further, the second unit configuration body 58, which is the zoom optical system, is moved toward a near side (in Tele (magnification) position direction) according to operation where the operation wire 66 is pulled in a near side direction thereof.

A guide space 67 for zoom guide which guides the action of movement of the projecting portion 65 of the second lens frame 58a in a zooming action direction is formed in the third lens frame 59a. A positioning member 68 for positioning a moving end of the projecting portion 65 of the second lens frame 58a when the projecting portion 65 moves in the Wide (wide-angle) position direction is provided at a distal end portion of the guide space 67. A striking portion 68a which abuts on a front end portion 65a of the projecting portion 65 of the second lens frame 58a to restrict a limit position of the front end portion 65a in the Wide (wide-angle) position direction is formed on the positioning member 68. A striking position between the striking portion 68a of the positioning member 68 and the front end portion 65a of the projecting portion 65 is set near a point of action 65b of the projecting portion 65 of the second lens frame 58a, namely, at a position near a coupling portion between the projecting portion 65 and the operation wire 66.

Incidentally, a stopper 500 for position restriction of movement of the projecting portion 65 of the second lens frame 58a in the Tele (magnification) side direction is provided at a rear end portion of the guide space 67. The stopper 500 is screwed and fixed to a stopper receiver 501 and the maximum magnification on the Tele (magnification) side can be adjusted by adjusting the position of the screwing.

A brightness diaphragm 70 is provided at the second lens frame 58a in the second unit configuration body 58 for sliding zooming. The brightness diaphragm 70 is disposed on a front surface side of the first lens 62a held by the second lens frame 58a. The brightness diaphragm 70 is provided with an opening formed at a central portion of a light-blocking sheet for allowing transmission of light.

Further, the second electric part unit 56 is continuously provided at a rear end portion of the fourth unit configuration body 60. The second electric part unit 56 includes a second imaging element 73 such as a CCD or a CMOS and a second circuit board 74 like the first imaging unit 28. Further, a cover lens 75 is provided on a light receiving face side of a front surface of the second imaging element 73.

The cover lens 75 of the second electric part unit 56 is fixed in a state that it is provided in parallel to an objective lens at a rear end portion of the second lens unit 55, namely, the second lens 64b of the fourth unit configuration body 60. Thereby, the second lens unit 55 and the second electric part unit 56 are integrated so that an observation optical unit for ordinary observation is formed.

The second circuit board 74 includes electric parts and a wiring pattern, and it is connected with distal end portions of a plurality of signal lines of a signal cable 76 by soldering or the like. Further, each outer peripheral portion of the cover lens 75, the second imaging element 73, the second circuit board 74, and a distal end portion of the signal cable 76 are covered with insulating sealing resin or the like.

An optical image focused on the second imaging element 73 from the second lens unit 55 is photoelectrically converted to electrical image signals by the second imaging element 73 and the image signals are output to the second circuit board 74. Further, electrical signals of an optical image output from the second circuit board 74 are transmitted to a subsequent electrical device, described later, via the signal cable 76.

Further, the second electric part unit 56 of the observation optical unit is protruded behind the third hole portion 15a3 of the cylindrical member 15a to be disposed at a position at which it does not contact with the cylindrical member 15a. Thereby, since heats of two CCDs (the first imaging element 51 in the first imaging unit 28 and the second imaging element 73 in the second imaging unit 30) do not interfere with each other, heat generation of the CCDs can be suppressed. Therefore, an endoscope 2 that realizes a reduction in noise due to heat generation of CCDs can be obtained.

As shown in FIG. 1, the signal cable 54 of the first imaging unit 28 and the signal cable 76 of the second imaging unit 30 extend into the connector 14 through the insertion portion 11, the operation portion 12, and the universal cable 13. A relay board 86 is housed in the connector 14. Proximal end portions of the signal cables 54 and 76 are connected to the relay board 86. The signal cables 54 and 76 are connected to a common signal cable 87 in a selectively switchable manner by a relay board 86 within the connector 14.

Further, the relay board 86 of the connector 14 is connected to a control circuit 89, described later, within the processor 4 via the signal cable 87 in the connector 14 and a switching signal line 88 in the scope cable 8.

Illumination lens units 90 are respectively provided at three illumination windows, namely, a first illumination window 29, a second illumination window 31, and a third illumination window 32, arranged at the distal end portion 15 of the insertion portion 11. As shown in FIG. 2, each illumination lens unit 90 includes a plurality of illumination lenses 91 and a holding frame 92 holding the illumination lenses 91. Incidentally, the first illumination window 29 and the second illumination window 31 are shown in FIG. 2.

Further, the illumination lenses 91 of the respective illumination lens units 90 are inserted into front end portions of three hole portions, namely, the second hole portion 15a2, the fourth hole portion 15a4, and the fifth hole portion 15a5, of the eight hole portions 15a1 to 15a8 formed in the cylindrical member 15a of the distal end portion 15 from the distal end side. Here, fixation is made in a state that the front end portion of the illumination lens 91 of the first illumination window 29 has been projected forward beyond the position of the flat surface 25a of the projecting step portion 25. Further, the front end portion of the illumination lens 91 of the first illumination window 29 has been projected forward beyond the front end position of the first lens 41a of the first imaging unit 28.

Distal end portions of light guides 93 transmitting illumination light are inserted and fitted in rear end portions of the second hole portion 15a2, the fourth hole portion 15a4, and the fifth hole portion 15a5, respectively. The light guide 93 has a distal end portion applied with a cylindrical member 94 and is covered with an outer skin 95 bundling a plurality of fibers and a protective tube 502 which is a gore material.

The light guide 93 extends into the connector 14 through the insertion portion 11, the operation portion 12, and the universal cable 13. A proximal end portion 96 of the light guide 93 is connected to a light guide connector (not shown) projecting from the connector 14. The light guide connector is connected to the light source apparatus 3 in an attachable and detachable manner.

The light source apparatus 3 includes a lamp 97 emitting white light, a collimator lens 98 collimating the light of the lamp 97 to produce parallel beams, and a condenser lens 100 collecting transmission light of the collimator lens 98 and emitting the same to the proximal end portion 96 of the light guide 93. Incidentally, the light source apparatus 3 includes a light adjusting function (not shown) adjusting the brightness of illumination light from the lamp 97.

In the embodiment, the light guide 93 is branched, for example, within the operation portion 12 such that three divided guides are inserted and extended in the insertion portion 11. Distal end portions of the respective three divided light guides 93 are disposed near back surfaces of the respective illumination lenses 91 of three illumination windows provided in the distal end cover 24, namely, the first illumination window 29, the second illumination window 31, and the third illumination window 32 so as to face them, and are screwed into rear end portions of the second hole portion 15a2, the fourth hole portion 15a4, and the fifth hole portion 15a5 of the cylindrical member 15a, for example.

Illumination light from the lamp 97 of the light source apparatus 3 is emitted on the proximal end portion 96 of the light guides 93, and illumination light guided through the light guide 93 is emitted forward from the endoscope 2 through the respective illumination lenses 91 of the first illumination window 29, the second illumination window 31, and the third illumination window 32.

A distal end portion of a communication tube 105 communicating with the treatment tool insertion channel 33 is inserted and fitted in the sixth hole portion 15a6 formed in the cylindrical member 15a of the distal end portion 15 from the proximal end portion side. A proximal end portion of the communication tube 105 is projected behind the cylindrical member 15a and a distal end portion of the treatment tool insertion channel 33 is connected to the proximal end portion of the communication tube 105. A distal end of the treatment tool insertion channel 33 communicates with the distal end opening 33a of the distal end cover 24.

The treatment tool insertion channel 33 is branched near the proximal end of the insertion portion 11 and one of branched channels 33 is inserted up to a treatment tool insertion port (not shown) disposed in the operation portion 12. The other of the branched channels 33 communicates with a suction channel through the insertion portion 11 and the universal cable 13 and a proximal end of the suction channel is connected to suction means (not shown) via the connector 14.

A proximal end portion of the gas-feeding and water-feeding nozzle 34 is inserted in a front end portion of the seventh hole portion 15a7 formed in the cylindrical member 15a of the distal end portion 15. Further, a distal end portion of a communication tube 107 communicating with a gas-feeding and water-feeding pipeline 106 for the gas-feeding and water-feeding nozzle 34 is inserted and fitted in a rear end portion of the seventh hole portion 15a7. A proximal end portion of the communication tube 107 is projected rearward beyond the cylindrical member 15a and a distal end portion of the gas-feeding and water-feeding pipeline 106 is coupled to the proximal end portion of the communication tube 107. Incidentally, the communication tube 107 and the gas-feeding and water-feeding pipeline 106 are connected and fixed to each other by bobbin winding.

A proximal end portion of the gas-feeding and water-feeding pipeline 106 is coupled to a branch pipe 108. Here, branched end portions 108a and 108b of the branch pipe 108 are connected with distal end portions of a gas-feeding pipeline 106a and a water-feeding pipeline 106b, respectively. Thereby, the gas-feeding and water-feeding pipeline 106 communicates with the gas-feeding pipeline 106a and the water-feeding pipeline 106b. Incidentally, the respective pipelines 106, 106a, and 106b, and the branch pipe 108 are connected and fixed by bobbin winding, and all peripheries of respective connection portions and the branch pipe 108 are applied with adhesive or the like so that the respective connection portions are kept air-tight (water-tight).

The gas-feeding pipeline 106a and the water-feeding pipeline 106b communicating with the gas-feeding and water-feeding nozzle 34 extend up to the connector 14 of the universal cable 13 to be connected to the gas-feeding and water-feeding apparatus 6 with a built-in pump (not shown) for performing gas feeding and water feeding.

A gas-feeding and water-feeding button 109 arranged in the operation portion 12 is interposed in midway portions of the gas-feeding pipeline 106a and the water-feeding pipeline 106b. Gas feeding and water feeding are performed according to operation of the gas-feeding and water-feeding button 109.

Thereby, a gas such as air or liquid such as sterile water is jetted from the jetting port 34a of the gas-feeding and water-feeding nozzle 34 in a jetting direction. At this time, a fluid such as sterile water or air jetted from the jetting port 34a of the gas-feeding and water-feeding nozzle 34 is guided to the first lens 61a of the second imaging unit 30 along the fluid guide face 26c to remove dirty body fluid or extraneous matter attached to a surface of the first lens 61a of the second imaging lens 30 and clean the surface, thereby allowing imaging in the observation viewing field in a clean state.

The forward water-feeding pipeline 35 extends up to the connector 14 through the insertion portion 11, the operation portion 12, and the universal cable 13 to be connected to the forward water-feeding apparatus 7. A forward water-feeding button (not shown) is interposed in the operation portion 12 in a midway portion of the forward water-feeding pipeline 35.

A liquid such as sterile water is sprayed in an insertion direction from the opening 35a of the distal end cover 24 of the insertion portion 11 to a body cavity according to operation of the forward water-feeding button. Thereby, body liquid present at the site to be examined within the body cavity or the like can be cleaned away. Incidentally, as shown in FIG. 1, a cable extending from the forward water-feeding apparatus 7 is connected with a foot switch 7a, so that a liquid such as sterile water can be sprayed in the insertion direction to the body cavity from the distal end face of the insertion portion 11.

Two drive circuits 110a and 110b, a signal processing circuit 111, and a control circuit 89 are provided within the processor 4. The drive circuits 110a and 110b drive the first imaging element 51 of the first imaging unit 28 and the second imaging element 73 of the second imaging unit 30, respectively. The signal processing circuit 111 performs signal processing on imaging signals output from the two imaging elements 51 and 73 via the relay board 86, respectively. The control circuit 89 controls an operating state of the signal processing circuit 111 and the like.

Control switches 112a and 112b, a gas-feeding and a water-feeding button 109, a bending operation knob (not shown), a zoom lever (not shown) performing zoom operation of the second imaging unit 30 for ordinary observation, a forward water-feeding button (not shown), and the abovementioned treatment tool insertion port (not shown) are provided in the operation portion 12 of the endoscope 2.

The control switches 112a and 112b are connected to the control circuit 89 of the processor 4 through signal lines 113a and 113b, respectively. In the embodiment, for example, the control switch 112a generates a signal instructing switching, and generates, for example, a signal for freeze instruction.

The relay board 86, for example, performs a switching action from a state that one of the signal cables 54 and 76 connected to the respective imaging elements 51 and 73 has been connected to the common signal cable 87 to a state that the other signal cable is connected to the signal cable 87 according to operation of the control switch 112a.

Specifically, for example, a switching signal is output from the control circuit 89 to the relay board 86 via the switching signal line 88 within the scope cable 8 according to operation of the control switch 112a. The relay board 86 is ordinarily in such a state that its input terminal for a signal from the control circuit 89 is at the L (LOW) level, which results in pull-down of a switching control terminal. In this state, the signal cable 54 of the second imaging unit 30 for ordinary observation is connected to the common signal cable 87. The switching control terminal is at the L level even in an activation state. That is, unless operation for a switching instruction is performed, setting to an ordinary observation state is maintained.

When a user operates the control switch 112a in this state, a signal from the control circuit 89 is applied to an input terminal of the relay board 86 via the switching signal line 88 as a control signal switching the input terminal to the H (HIGH) level, resulting in pull-up of the switching control terminal. In this state, the signal cable 54 of the first imaging unit 28 of an object contact type is connected to the common signal cable 87.

Further, when the control switch 112a is operated, a signal of level L is supplied to the switching control terminal so that the signal cable 54 of the second imaging unit 30 for ordinary observation is connected to the common signal cable 87.

The control circuit 89 controls an action state of the signal processing circuit 111 so as to correspond to the imaging element 51 of the second imaging unit 30 for ordinary observation and the imaging element 73 of the first imaging unit 28 of an object contact type according to operation of the control switch 112a.

Each endoscope image of the first imaging unit 28 of an object contact type or the second imaging unit 30 for ordinary observation is displayed on the monitor 5 according to input of a video signal output from the signal processing circuit 111 of the processor 4.

A subject image photographed by each of two imaging units 28 and 30 is displayed on the monitor 5 (see FIG. 1), where upward and downward directions of the monitor 5 coincide with a vertical transfer direction of a CCD element or a CMOS element of each of the imaging elements 51 and 73, while leftward and rightward directions thereof coincide with a horizontal transfer direction of the CCD element or the CMOS element of each of the imaging elements 51 and 73. That is, the upward, downward, leftward, and rightward directions of an endoscope image obtained by each of the two imaging units 28 and 30 coincide with the upward, downward, leftward, and rightward directions of the monitor 5.

Upward, downward, leftward, and rightward directions of the bending portion 16 of the insertion portion 11 are determined so as to correspond to upward, downward, leftward, and rightward directions of an endoscope image displayed on the monitor 5. That is, four bending operation wires extending in the bending portion 16 are pulled or loosened according to predetermined operations of the bending operation knob provided on the operation portion 12, so that the bending portion 16 can be freely bent in four directions of upward, downward, leftward and rightward, corresponding to upward, downward, leftward, and rightward directions of an image displayed on the monitor 5.

That is, two imaging units 28 and 30 are determined such that a horizontal transfer direction and a vertical transfer direction of the imaging elements 51 and 73 correspond to installation directions of the imaging elements 51 and 73 within the distal end portion 15, respectively, such that, even if switching between ordinary observation and magnification observation of an object contact type is performed, upward, downward, leftward, and rightward directions of an endoscope image displayed on the monitor 5 are always equal to upward, downward, leftward, and rightward directions of bending operation directions of the bending portion 16.

Thereby, a user can perform bending operations in upward, downward, leftward, and rightward directions of the bending portion 16 without feeling discomfort in upward, downward, leftward, and rightward directions of an endoscope image displayed on the monitor 5 when he/she switches the endoscope image between an ordinary observation image and a magnified observation image.

As shown in FIG. 2, a distal end hood 201 is attached on an outer peripheral surface of the distal end cover 24 of the insertion portion 11. The distal end hood 201 includes a cylindrical hood main body 202. A mounting portion 203 mounted on an outer peripheral surface of the distal end cover 24 in a fitted state is provided on a proximal end portion of the hood main body 202. An elastically deformable elastic deformation portion 204 is disposed at a distal end portion of the hood main body 202. Here, the hood main body 202 is made from a relatively hard resin material, for example, transparent acrylic elastomer or black polysulfone. The elastic deformation portion 204 is made from a resin material or rubber material softer than the hood main body 202, for example, silicon rubber.

The distal end portion of the hood main body 202 is provided so as to extend slightly forward beyond a position corresponding to the flat surface 26a of the middle step portion 26 of the distal end cover 24. The elastic deformation portion 204 is provided so as to extend rearward beyond a position corresponding to the flat surface 25a of the projecting step portion 25 of the distal end cover 24.

The elastic deformation portion 204 is configured such that the distal end portion of the elastic deformation portion 204 abuts on an analyte H such as a body tissue to position an observation position of the second imaging unit 30 at a magnification observation time utilizing the second imaging unit 30. Here, as shown in FIG. 2, the distal end portion of the elastic deformation portion 204 is set so as to be disposed in an observation depth range A between a depth of field L1 on a far point side of the second imaging unit 30 and a depth of field L2 on a near point side thereof at the magnification observation time utilizing the second imaging unit 30. Incidentally, the depth of field L1 on the far point side of the second imaging unit 30 is set to, for example, 2.5 mm, while the depth of field L2 on the near point side is set to, for example, 2.0 mm.

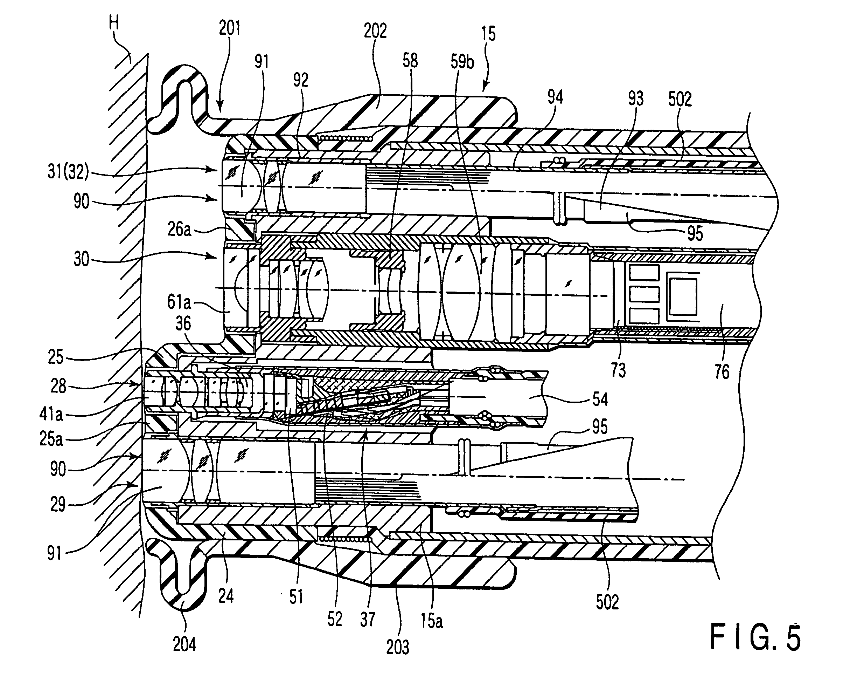

Further, the elastic deformation portion 204 is set such that, when the elastic deformation portion 204 is pressed on an analyte H with a further large pressing force for pressing the elastic deformation portion 204 to the analyte H after the distal end portion of the elastic deformation portion 204 abuts on the analyte H, the elastic deformation portion 204 is shrunk and deformed in a striking direction, as shown in FIG. 5. At this time, the elastic deformation portion 204 is set such that the elastic deformation portion is elastically deformable up to a position at which the first imaging unit 28 abuts on the analyte H at a contact observation time utilizing the first imaging unit 28. Further, the elastic deformation portion 204 is elastically deformed to be evacuated out of a field of view of the first lens 41a of the first imaging unit 28 due to shrinking deformation to a state that an outer peripheral surface of the hood main body 202 bulges outwardly in an approximate U shape at an elastic deformation time of the elastic deformation portion 204.

Next, an operation of the endoscope system 1 with the abovementioned configuration will be explained.

The endoscope system 1 is set as shown in FIG. 1 at a use time of the endoscope 2 according to the embodiment. That is, a user connects the connector 14 of the endoscope 2 to the light source apparatus 3, further connects one end of the scope cable 8 to the connector 14, and connects the other end of the scope cable 8 to the processor 4. The user connects the gas-feeding pipeline 106a and the water-feeding pipeline 106b to the gas-feeding and water-feeding apparatus 6.

The user turns ON power switches of the light source apparatus 3, the processor 4, and the like to set these apparatuses to their serving states. At this time, the control circuit 89 of the processor 4 can perform transmission and reception of a control signal and the like.

In the activated state, the relay board 86 is set such that the second imaging unit 30 for ordinary observation is selected. At this time, the control circuit 89 performs control so as to drive the drive circuit 110b and sets the serving state of the signal processing circuit 111 to an observation mode for ordinary observation.

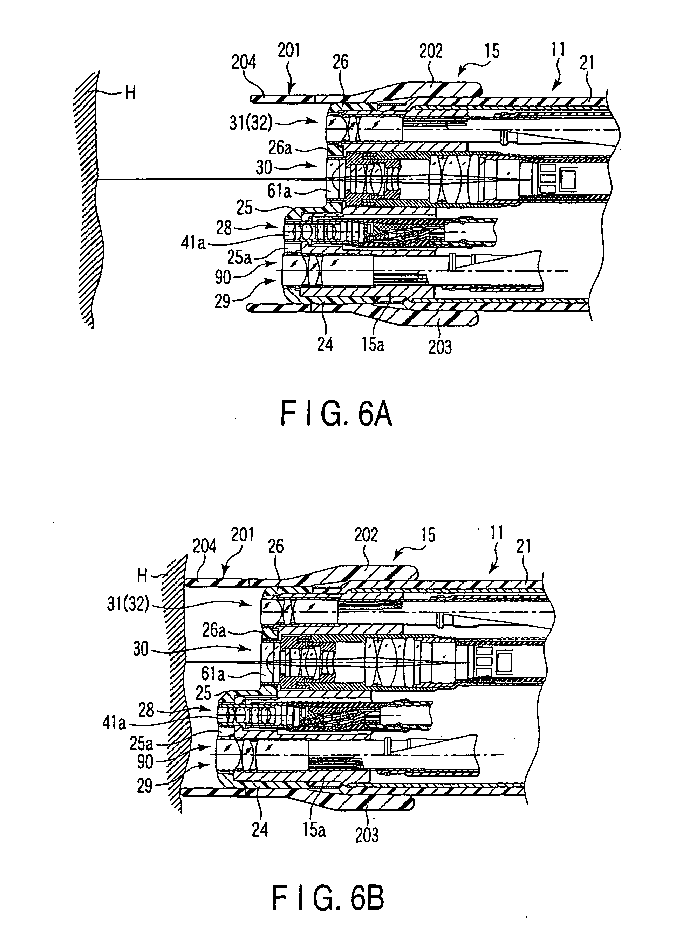

After the setting of the endoscope system 1 is completed, the task of inserting the endoscope 2 into a body of a patient is started. At the insertion time of the endoscope 2, the user inserts the insertion portion 11 of the endoscope 2 into a body cavity and performs setting so as to be capable of observing an affected area to be diagnosed or the like. At this time, the operation lever for zooming (not shown) of the operation portion 12 is maintained at the Wide (wide-angle) position. Thereby, as shown in FIG. 6A, the second unit configuration body 58, which is the zoom optical system, is held in a state that it has been moved forward (in the Wide (wide-angle) position direction).

The light source apparatus 3 is put in an illumination light supply state. Illumination light beams of R, G and B, for example, are supplied to the light guide 93 in a sequential-frame manner. In synchronization therewith, the drive circuit 110b outputs a CCD drive signal to illuminate the affected area or the like within the body cavity through the first illumination window 29 and the second and third illumination windows 31 and 31.

A subject of the illuminated affected area or the like is focused on the light receiving face of the second imaging element 73 through the second lens unit 55 of the second imaging unit 30 for ordinary observation to be photoelectrically converted. The second imaging element 73 outputs a photoelectrically converted signal according to application of a drive signal. The converted signal is input into the signal processing circuit 111 via the signal cable 76 and the common signal cable 87 selected by the relay board 86. The signal input into the signal processing circuit 111 is A/D-converted within the signal processing circuit 111 to be temporarily stored in memories for R, G, and B.

Thereafter, signals stored in the memories for R, G, and B are simultaneously read to form synchronized R, G, and B signals and further D/A converted to form analog R, G and B signals, so that color display is performed on the monitor 5. Thereby, ordinary observation for observing an object to be observed separated from the first lens 61a of the second imaging unit 30 over a wide range is performed using the second imaging unit 30 for ordinary observation.

When dirt such as body fluid or extraneous matter has attached to a surface of the first lens 61a of the second imaging unit 30 during ordinary observation, the gas-feeding and water-feeding button 109 is operated. Gas feeding and water feeding are performed through the gas-feeding pipeline 106a and the water-feeding pipeline 106b according to the operation of the gas-feeding and water-feeding button 109. Gas such as air or a liquid such as sterile water is jetted in the jetting direction from the jetting port 34a of the gas-feeding and water-feeding nozzle 34 on the lower step portion 27 of the projecting step portion 25. At this time, a fluid such as sterile water or air jetted from the jetting port 34a of the gas-feeding and water-feeding nozzle 34 is guided to the first lens 61a of the second imaging unit 30 along the fluid guide face 26c of the projecting step portion 25 so that the dirt such as body fluid or extraneous matter which has attached to a surface of the first lens 61a of the second imaging unit 30 is removed and the surface is cleaned, and imaging and an observation field of view in a clean state can be ensured.

Further, when a site to be examined within the body cavity has become dirty due to adhesion of body fluid or the like, the forward water-feeding button is operated. A liquid such as sterile water is sprayed from the opening 35a of the distal end cover 24 of the insertion portion 11 toward the insertion direction to the body cavity at the operation time of the forward water-feeding button. Thereby, body fluid which has attached to the site to be examined within the body cavity or the like can be cleaned away.

The observation utilizing the second imaging unit 30 for ordinary observation is continued until the distal end portion of the endoscope 2 inserted into the body of the patient reaches the target site to be observed. In a state that the distal end portion of the endoscope 2 has approached the target site to be observed, the distal end portion of the distal end hood 201 is pushed on the analyte H in a state that it surrounds the target site to be observed, as shown in FIG. 6B. Thereby, the observation position of the second imaging unit 30 is positioned at the magnification observation time utilizing the second imaging unit 30. The distal end portion of the elastic deformation portion 204 is disposed in the observation depth range A between the depth of field L1 on the far point side of the second imaging unit 30 and the depth of field L2 on the near point side at the magnification observation time utilizing the second imaging unit 30.

In this state, the operation lever for zooming (not shown) on the operation portion 12 is operated in the Tele (magnification) position direction by a user. Thereby, the operation wire 66 is pulled toward the near direction so that the second unit configuration body 58, which is the zoom optical system, is moved toward the near side (in Tele (magnification) position direction). In this state, magnification observation utilizing the second imaging unit 30 is performed.

Further, after the magnification observation utilizing the second imaging unit 30, when switching to an object contact observation utilizing the first observation unit 28 is performed, the control switch 112a is turned ON. At the ON operation time of the control switch 112a, the control circuit 89 receives a switching instruction signal to perform switching control to the relay board 86. At this time, the control circuit 89 controls the drive circuit 110b to a serving state and sets the signal processing circuit 111 to an observation mode of high power. Thereby, switching from the ordinary observation mode utilizing the second imaging unit 30 to the observation mode of high power using the first imaging unit 28 of an object contact type is performed.

Thus, the distal end portion of the first lens 41a of the first imaging unit 28 is brought into contact with an object in a state that switching to the observation mode of high power has been switched, so that an object contact observation of high power where a cell tissue to be observed or the like is observed with high power, or the like is performed. Incidentally, when magnification observation of high power is performed, dye is preliminarily sprayed on a site of interest and the site of interest is dyed so that a contour of a cell can be observed distinctly.

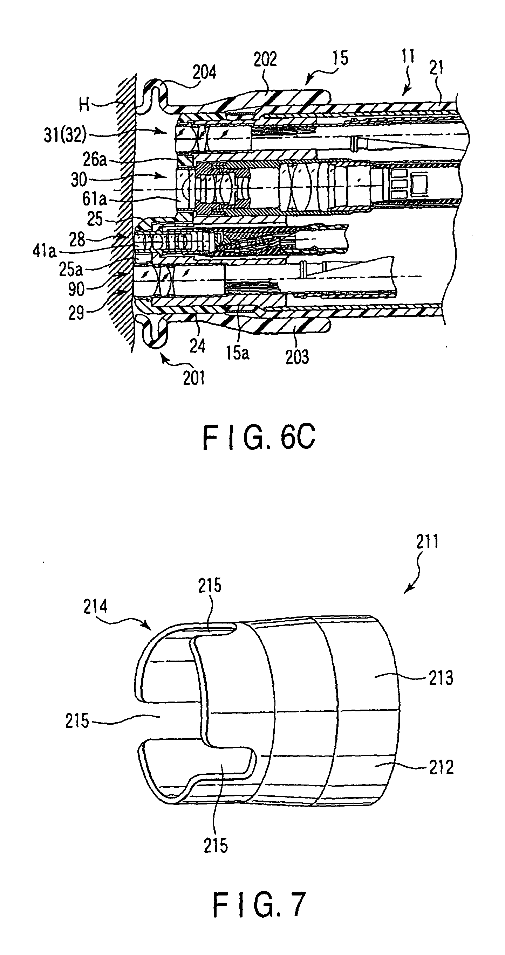

At an observation time of a body tissue of analyte H utilizing the first imaging unit 28 of a object contact type, a pressing force for pressing the distal end portion of the elastic deformation portion 204 of the distal end hood 201 to an analyte H is made larger than that at the magnification observation time utilizing the second imaging unit 30. Thereby, the elastic deformation portion 204 of the distal end hood 201 is shrunk and deformed in the striking direction. At this time, the elastic deformation portion 204 is shrunk and deformed to a state that an outer peripheral surface side of the hood main body 202 bulges outwardly in an approximate U shape, so that the elastic deformation portion 204 is elastically deformed so as to be evacuated out of the field of view of the first lens 41a of the first imaging unit 28. Thereby, as shown in FIG. 6C, the projecting step portion 25 of the distal end cover 24 is mainly pressed onto a surface of the body tissue of the analyte H and the non-projecting face other than the projecting step portion 25 is kept in a non-contacting state with the surface of the body tissue of the analyte H. Therefore, the first lens 41a at the distal end of the first imaging unit 28 and the illumination lens 91 of the first illumination window 29, which are disposed on the projecting step portion 25, are brought into contact with the surface of the body tissue of the analyte H such as a cell tissue to be observed. Incidentally, the observation range of the first imaging unit 28 of super high power is shallow in observation depth, such as a range from the first lens 41a, which is the observation window, of 0 to 100µ, which easily results in an unstable observation state due to jiggling or blurring. Therefore, when observation is performed using the first imaging unit 28 of super high power, the observation is performed in a state that the first lens 41a, which is the observation window, is brought into contact with an analyte so that the endoscope distal

end portion 15 is held in a non-jiggling state.

In this state, illumination light is emitted to a body tissue of the analyte H such as a cell tissue through the illumination lens 91 of the first illumination window 29. At this time, a portion of the illumination light emitted to the body tissue of the analyte H such as a cell tissue permeates the body tissue of the analyte H such as a cell tissue to spread over the striking face of the illumination lens 91 of the first illumination window 29. Therefore, a surrounding portion of the body tissue of the analyte H such as a cell tissue ahead of the first lens 41a of the first imaging unit 28 is also irradiated with illumination light. Thereby, a portion to be observed by the first lens 41a of the first imaging unit 28 pressed on a surface of the body tissue of the analyte H such as a cell tissue is also irradiated with illumination light so that light of the body tissue of the analyte H such as a cell tissue is focused on the light receiving face of the first imaging element 51 through the lens unit 36 of the first imaging unit 28 to be photoelectrically converted.

The first imaging element 51 outputs the photoelectrically converted signal according to application of a drive signal from the drive circuit 110b. In this case, the signal is amplified within the first imaging element 51 to be output from the first imaging element 51. The signal is input into the signal processing circuit 111 through the signal cable 54 and the common signal cable 87 selected by the relay board 86.

After the signal input to the signal processing circuit 111 is A/D-converted, the A/D-converted signals are simultaneously stored in memories for R, G, and B, for example. Thereafter, signals stored in the memories for R, G, and B are simultaneously read to form synchronized R, G, and B signals, and further D/A converted to form analog R, G, and B signals, so that display on the monitor 5 is performed. Thereby, observation of a body tissue of an analyte H such as a cell tissue ahead of the first lens 41a of the first imaging unit 28 is performed with an observation mode of high power using the first imaging unit 28 of an object contact type.

Therefore, the following effects can be achieved with the abovementioned configuration. That is, according to the embodiment, the elastic deformation portion 204 is disposed at the distal end portion of the hood main body 202 of the distal end hood 201 attached on the outer peripheral surface of the distal end cover 24 of the insertion portion 11. At the magnification observation time where an analyte H is observed in a magnified manner in a non-contact state of the second imaging unit 30 with the analyte H, the observation position of the second imaging unit 30 can be positioned by causing the distal end portion of the elastic deformation portion 204 of the hood main body 202 to abut on the analyte H. Thereby, even in a state that the observation depth range A is shallow (0.5 mm or so) at the magnification observation (zoom) time utilizing the first lens 61a, which is the observation lens of the second imaging unit 30, the observation position of the second imaging unit 30 can be positioned at a proper position for performing magnification observation utilizing the first lens 61a, which is the observation lens of the second imaging unit 30. Therefore, the magnification observation (zoom) utilizing the first lens 61a, which is the observation lens of the second imaging unit 30, can be performed stably so that a proper observation image can be obtained.

At the contact observation time utilizing the first imaging unit 28, the elastic deformation portion 204 of the hood main body 202 can be elastically deformed up to a position at which the first lens 41a, which is the observation lens of the first imaging unit 28, abuts on an analyte H. Thereby, since the first lens 41a, which is the observation lens of the first imaging unit 28, can be brought into contact with the analyte H stably, contact observation of a body tissue of the analyte H such as a cell tissue ahead of the first lens 41a of the first imaging unit 28 can be performed stably, and a proper contact observation image can be obtained in the observation mode of high power using the first imaging unit 28 of an object contact type. Accordingly, in the embodiment, such an effect can be obtained that the magnification (zoom) observation of a surface of a body tissue utilizing the second imaging unit 30, which is the ordinary observation optical system, can be performed stably even by the endoscope 2 provided with the first imaging unit 28, which is the observation optical system of an object contact type, and which is brought into contact with a body tissue to observe the same, and object contact observation which brings the first imaging unit 28 of an object contact type into contact with a body tissue to observe the same can also be performed stably.

FIG. 7 to FIG. 9 show a second embodiment of the present invention. This embodiment has a configuration in which the distal end hood 201 of the endoscope 2 of the endoscope system 1 according to the first embodiment (see FIG. 1 to FIG. 6C) has been modified in the following manner.

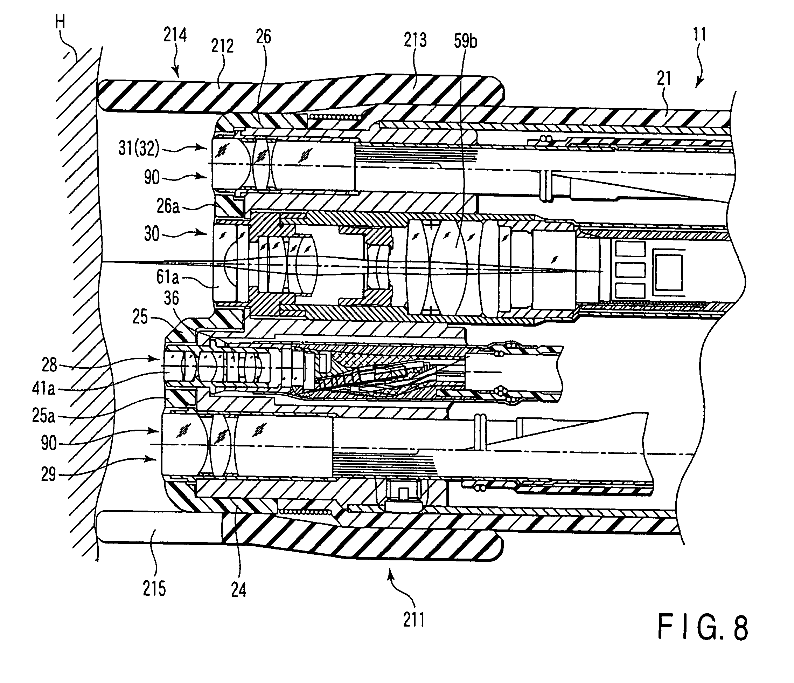

That is, in a distal end hood 211 of the endoscope 2 according to the embodiment, a hood main body 212 has a configuration in which a mounting portion 213 mounted on an outer peripheral surface of a distal end portion 15 and an elastic deformation portion 214 are made of the same material. Further, the elastic deformation portion 214 has such a configuration that a plurality of, three in this embodiment, U-shaped groove portions (notch portions) 215 notched in an approximate U shape are formed at a distal end portion of a cylindrical wall portion of the hood main body 212 so that flexibility of the wall portion is made larger than that of the mounting portion 213. As shown in FIG. 8, groove bottom portions of three U-shaped groove portions 215 are provided to extend rearward beyond a position corresponding to the flat surface 25a of the projecting step portion 25 of the distal end cover 24. Incidentally, the portions other than the U-shaped portions in this embodiment are identical in configuration to those of the endoscope 2 according to the first embodiment, where same portions as those in the endoscope 2 according to the first embodiment are attached with same reference numerals and explanation thereof is omitted.

In this embodiment, at the magnification observation time of the zoom optical system utilizing the second imaging unit 30 for ordinary observation, an observation position at the magnification observation time is positioned by causing the distal end portion of the electrically deformable portion 214 to abut on an analyte H, as shown in FIG. 8. At the magnification observation time utilizing the second imaging unit 30, the distal end portion of the elastic deformation portion 214 is disposed in the observation depth range A between the depth of field L1 on the far point side of the second imaging unit 30 and the depth of field L2 on the near point side. In this state, the magnification observation utilizing the second imaging unit 30 is performed.

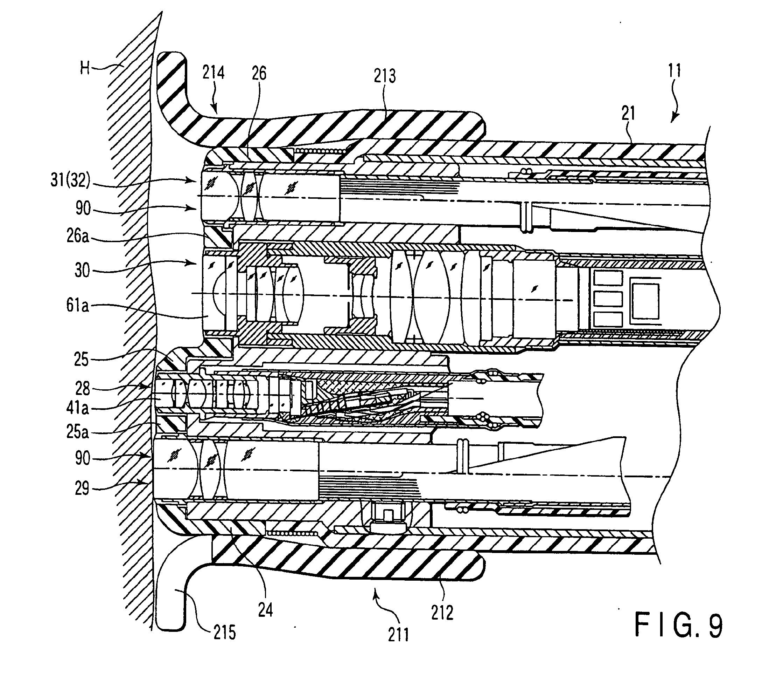

At an observation time of a body tissue of an analyte H utilizing the first imaging unit 28 of an object contact type, a pressing force for pressing the distal end portion of the elastic deformation portion 214 of the distal end hood 211 to the analyte H is made larger than that at the magnification observation time utilizing the second imaging unit 30. Thereby, the elastic deformation portion 214 of the distal end hood 211 is shrunk and deformed in the striking direction. At this time, as shown in FIG. 9, the elastic deformation portion 214 is shrunk and deformed to a state in which an outer peripheral surface side of the hood main body 212 is bent outwardly in an approximate L shape, so that the elastic deformation portion 214 is elastically deformed to be evacuated out of the field of view of the first lens 41a of the first imaging unit 28. Thereby, as shown in FIG. 9, the projecting step portion 25 of the distal end cover 24 is mainly pressed onto a surface of the body tissue of the analyte H and the non-projecting face other than the projecting step portion 25 is kept in a non-contact state with the surface of the body tissue of the analyte H.

Therefore, the first lens 41a at the distal end of the first imaging unit 28 and the illumination lens 91 of the first illumination window 29 which are disposed on the projecting step portion 25 are brought into contact with the surface of the body tissue of the analyte H, such as a cell tissue to be observed. Thereby, the object contact observation of high power where the distal end portion of the first lens 41a of the first imaging unit 28 is brought into contact with an object so that a cell tissue to be observed or the like is observed with high power, or the like is performed.

Therefore, the following effects can be achieved with the abovementioned configuration. That is, according to the embodiment, the elastic deformation portion 214 is provided, and is constituted of a plurality of, three in this embodiment, U-shaped groove portions (notch portions) 215 notched in an approximate U shape and formed at a distal end portion of a cylindrical wall portion of the hood main body 212 so that the flexibility of the wall portion is made larger than that of the mounting portion 213. Thereby, like the first embodiment, such an effect can be obtained even in the distal end hood 211 according to this embodiment that the magnification (zoom) observation of a surface of a body tissue utilizing the second imaging unit 30, which is an ordinary observation optical system, can be performed stably even by the endoscope 2 provided with the first imaging unit 28, which is the observation optical system of an object contact type and which is brought into contact with a body tissue to observe the same, and object contact observation which brings the first imaging unit 28 of an object contact type in contact with a body tissue to observe the same can also be performed stably.

Further, in the distal end hood 211 according to the embodiment, since the mounting portion 213 mounted to the outer peripheral surface of the distal end portion 15 and the elastic deformation portion 214 can be made of the same material, the manufacturing cost of the hood main body 212 can be reduced.

Further, in the distal end hood 211 according to the embodiment, water accumulated inside the distal end hood 211 can be caused to flow outside from the U-shaped groove portions 215 of the distal end hood 211. Therefore, an adverse effect on observation due to water accumulating inside the distal end hood 211 can be reduced.

Incidentally, such a configuration can be adopted that a thin portion, thinner in wall portion thickness than that of the mounting portion 213 mounted to the outer peripheral surface of the distal end portion 15, is formed at the distal end portion of the cylindrical wall portion of the hood main body 212, and the elastic deformation portion 214 is made of the thin portion.

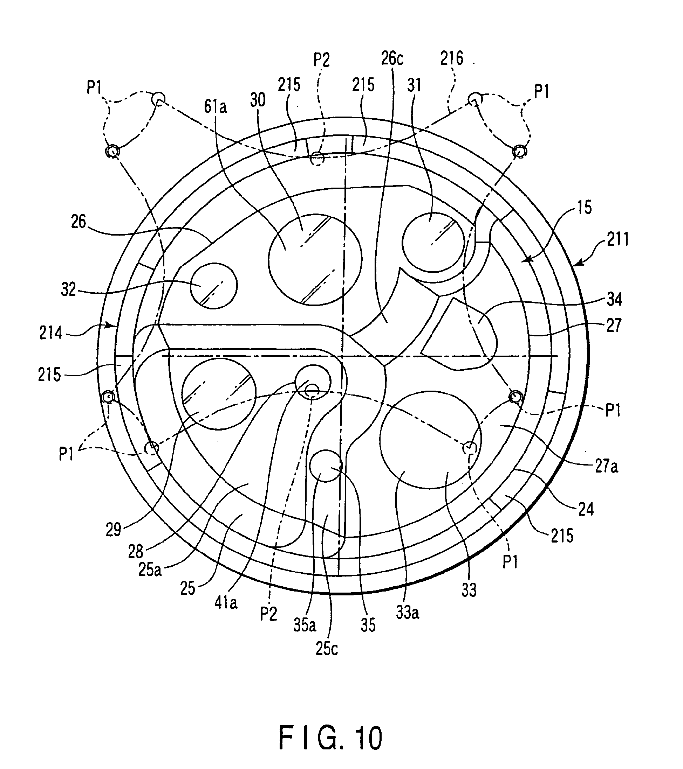

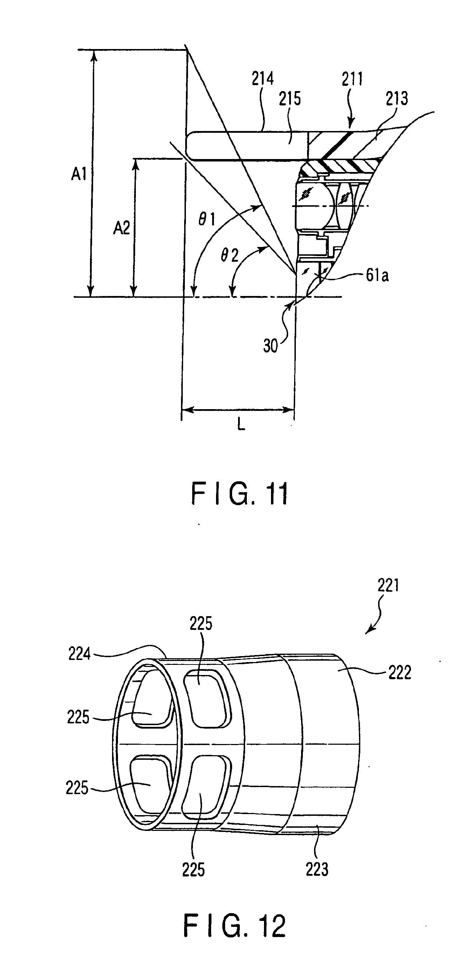

FIG. 10 and FIG. 11 show a modified embodiment of the distal end hood 211 according to the second embodiment. In the modified embodiment, a configuration of the elastic deformation portion 214 of the distal end hood 211 has been modified in the following manner.

That is, as shown in FIG. 10, in an endoscope 2 according to this embodiment, such a configuration is adopted that four U-shaped groove portions 215 are formed at a distal end portion of the cylindrical wall portion of the hood main body 212 and these U-shaped groove portions 215 are arranged in the cylindrical wall portion of the hood main body 212 corresponding to a field of view area 216 of the first lens 61a, which is the observation lens of the second imaging lens 30 for ordinary observation. Incidentally, in FIG. 10, P1 and P2 indicate a field angle maximum position and a field angle minimum position in the view area 216 of the first lens 61a at a position (a position of the distal end portion of the elastic deformation portion 204) apart from a surface of the first lens 61a of the second imaging unit 30 by a distance L, respectively.

FIG. 11 shows a maximum field angle θ1 and a minimum field angle θ2 in the view area 216 at a position (a position of the distal end portion of the elastic deformation portion 204) apart from a surface of the first lens 61a, which is the observation lens of the second imaging unit 30 for ordinary observation, by a distance L. In FIG. 11, A1 and A2 indicate a view range in the maximum field angle direction and a view range in the minimum field angle direction, respectively.

Therefore, the following effects can be achieved in the modified embodiment with the abovementioned configuration. That is, according to the modified embodiment, since the U-shaped groove portions 215 are disposed in the portion of the cylindrical wall portion of the hood main body 212 corresponding to the view area 216 of the first lens 61a, which is the observation lens of the second imaging unit 30, such a phenomenon that the view of the first lens 61a, which is the observation lens of the second imaging unit 30, is interrupted by the cylindrical wall portion of the hood main body 212, so-called "view vignetting" can be prevented. Therefore, a sufficiently wide view area 216 can be ensured even at the ordinary observation time utilizing the first lens 61a, which is the observation lens of the second imaging unit 30.

FIG. 12 shows a third embodiment of the present invention. This embodiment has such a configuration in which the distal end hood 201 of the endoscope 2 of the endoscope system 1 according to the first embodiment (see FIG. 1 to FIG. 6C) has been modified in the following manner.

That is, in a distal end hood 221 of an endoscope 2 according to this embodiment, a hood main body 222 has such a configuration that a mounting portion 223 mounted on an outer peripheral surface of a distal end portion 15 and an elastic deformation portion 224 are made of the same material. Further, the elastic deformation portion 224 has such a configuration that a plurality of, four in this embodiment, approximately rectangular openings 225 are formed at a distal end portion of a cylindrical wall portion of the hood main body 222 so that flexibility of the wall portion is made larger than that of the mounting portion 213. Incidentally, the portions other than the rectangular openings in this embodiment are identical in configuration to those of the endoscope 2 according to the first embodiment, where same portions as those in the endoscope 2 according to the first embodiment are attached with same reference numerals and explanation thereof is omitted.

In this embodiment, at the magnifiaction observation time of the zoom optical system utilizing the second imaging unit 30 for ordinary observation, an observation position at the magnification observation time is positioned by causing the distal end portion of the electrically deformable portion 224 to abut on an analyte H. At the magnification observation time utilizing the second imaging unit 30, the distal end portion of the elastic deformation portion 224 is disposed in the observation depth range A between the depth of field L1 on the far point side of the second imaging unit 30 and the depth of field L2 on the near point side. In this state, the magnfication observation utilizing the second imaging unit 30 is performed.

At an observation time of a body tissue of an analyte H utilizing the first imaging unit 28 of an object contact type, a pressing force for pressing the distal end portion of the elastic deformation portion 224 of the distal end hood 221 to the analyte H is made further larger than that at the magnification observation time utilizing the second imaging unit 30. Thereby, the elastic deformation portion 224 of the distal end hood 221 is shrunk and deformed in the striking direction. At this time, the elastic deformation portion 224 is shrunk and deformed in the axial direction to a state that an outer peripheral surface side of the hood main body 222 bulges outwardly in an approximately mountain shape, so that the elastic deformation portion 224 is elastically deformed so as to be evacuated out of the field of view of the first lens 41a of the first imaging unit 28. Thereby, the projecting step portion 25 of the distal end cover 24 is mainly pressed on a surface of the body tissue of the analyte H and the non-projecting face other than the projecting step portion 25 is kept in non-contacting state with the surface of the body tissue of the analyte H. Therefore, the first lens 41a at the distal end of the first imaging unit 28 and the illumination lens 91 of the first illumination window 29 which are disposed on the projecting step portion 25 are brought into contact with the surface of the body tissue of the analyte H such as a cell tissue to be observed. Thereby, the object contact observation of high power where the distal end portion of the first lens 41a of the first imaging unit 28 is brought into contact with an object so that a cell tissue to be observed or the like is observed with high power, or the like is performed.

Therefore, the following effects can be achieved with the abovementioned configuration. That is, according to the embodiment, the elastic deformation portion 224 where four approximately rectangular openings 225 are formed at a distal end portion of a cylindrical wall portion of the hood main body 222 so that flexibility of the wall portion is made larger than that of the mounting portion 223 is provided. Thereby, like the first embodiment, such an effect can be obtained even in the distal end hood 221 according to this embodiment that the magnification (zoom) observation of a surface of a body tissue utilizing the second imaging unit 30 which is the ordinary observation optical system can be performed stably even by the endoscope 2 provided with the first imaging unit 28 which is the observation optical system of an object contact type and which is brought into contact with a body tissue to observe the same and object contact observation which brings the first imaging unit 28 of an object contact type in contact with a body tissue to observe the same can also be performed stably.

Further, in the distal end hood 221 according to the embodiment, since the mounting portion 223 mounted to the outer peripheral surface of the distal end portion 15 and the elastic deformation portion 224 can be made of the same material, manufacturing cost of the hood main body 222 can be reduced.

Further, in the distal end hood 221 according to the embodiment, water accumulating inside the distal end hood 221 can be caused to flow outside from four openings 225 of the hood main body 222. Therefore, adverse effect to observation due to remaining water accumulating inside the distal end hood 221 can be reduced.

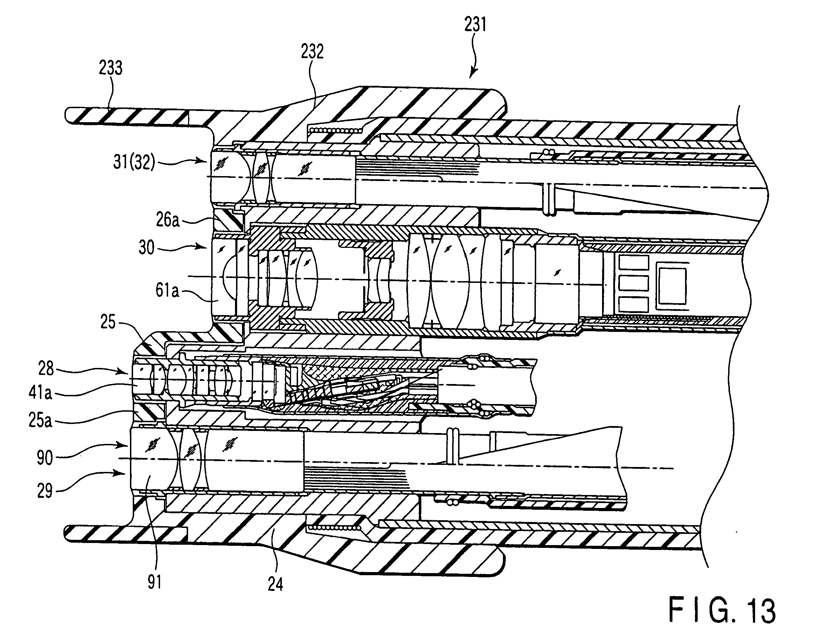

FIG. 13 shows a fourth embodiment of the present invention. This embodiment has such a configuration that the configuration of the endoscope 2 of the endoscope system 1 according to the first embodiment (see FIG. 1 to FIG. 6C) has been modified in the following manner.

That is, in the endoscope 2 according to the embodiment, a distal end cover 24 of the insertion portion 11 and a distal end hood 231 are formed integrally. Here, the distal end hood 231 includes a hood main body 232 integrated with the distal end cover 24 and made from relatively hard resin material and an elastic deformation portion 233 which is elastically deformable and is disposed at a distal end portion of the hood main body 232. The elastic deformation portion 233 is made of resin material or rubber material softer than the hood main body 232, for example, silicon rubber. The elastic deformation portion 233 is provided so as to extend rearward beyond a position corresponding to the flat surface 25a of the projecting step portion 25 of the distal end cover 24. Incidentally, portions other than the elastic deformation portions 233 in this embodiment are identical in configuration to those of the endoscope 2 according to the first embodiment, where same portions as those in the endoscope 2 according to the first embodiment are attached with same reference numerals and explanation thereof is omitted.

Even the endoscope 2 according to this embodiment can be used like the endoscope 2 according to the first embodiment. At the magnification observation time of the zoom optical system utilizing the second imaging unit 30 for ordinary observation, an observation position at the magnification observation time is positioned by causing the distal end portion of the electrically deformable portion 233 to abut on an analyte H. At the magnification observation time utilizing the second imaging unit 30, the distal end portion of the elastic deformation portion 233 is disposed in the observation depth range A between the depth of field L1 on the far point side of the second imaging unit 30 and the depth of field L2 on the near point side. In this state, the magnification observation utilizing the second imaging unit 30 is performed.

At an observation time of a body tissue of an analyte H utilizing the first imaging unit 28 of an object contact type, a pressing force for pressing the distal end portion of the elastic deformation portion 233 of the distal end hood 231 to the analyte H is made further larger than that at the magnification observation time utilizing the second imaging unit 30. Thereby, the elastic deformation portion 233 of the distal end hood 231 is shrunk and deformed in the striking direction. At this time, the elastic deformation portion 233 is shrunk and deformed to a state that an outer peripheral surface side of the hood main body 232 bulges outwardly in an approximately U shape, so that the elastic deformation portion 233 is elastically deformed so as to be evacuated out of the field of view of the first lens 41a of the first imaging unit 28. Thereby, the projecting step portion 25 of the distal end cover 24 is mainly pressed on a surface of the body tissue of the analyte H and the non-projecting face other than the projecting step portion 25 is kept in non-contacting state with the surface of the body tissue of the analyte H. Therefore, the first lens 41a at the distal end of the first imaging unit 28 and the illumination lens 91 of the first illumination window 29 which are disposed on the projecting step portion 25 are brought into contact with the surface of the body tissue of the analyte H such as a cell tissue to be observed. Thereby, the object contact observation of high power where the distal end portion of the first lens 41a of the first imaging unit 28 is brought into contact with an object so that a cell tissue to be observed or the like is observed with high power, or the like is performed.

Therefore, the following effects can be achieved with the abovementioned configuration. That is, according to the embodiment, the distal end cover 24 of the insertion portion 11 and the distal end hood 231 are formed integrally, and the hood main body 232 made of relatively hard resin material and integrated with the distal end cover 24 and the elastic deformation portion 233 elastically deformable and disposed at the distal end portion of the hood main body 232 are disposed on the distal end hood 231. Thereby, like the first embodiment, such an effect can be obtained even in the distal end hood 231 according to this embodiment that the magnification (zoom) observation of a surface of a body tissue utilizing the second imaging unit 30 which is the ordinary observation optical system can be performed stably even by the endoscope 2 provided with the first imaging unit 28 which is the observation optical system of an object contact type and which is brought into contact with a body tissue to observe the same and object contact observation which brings the first imaging unit 28 of an object contact type in contact with a body tissue to observe the same can also be performed stably.

Further, according to the embodiment, since the distal end cover 24 of the insertion portion 11 and the distal end hood 231 are formed integrally, the number of assembling steps and manufacturing cost can be reduced as compared with a case that a distal end hood prepared as a separate part is assembled to the distal end cover 24 of the insertion portion 11.

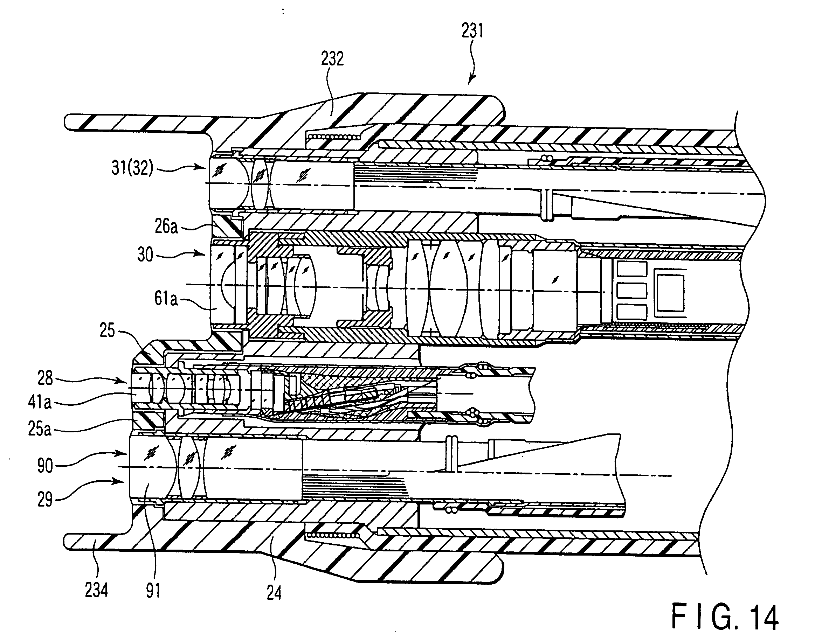

FIG. 14 shows a fifth embodiment of the present invention. This embodiment has such a configuration that the configuration of the distal end hood 231 of the endoscope 2 of the endoscope system 1 according to the fourth embodiment (see FIG. 13) has been modified in the following manner.

That is, in the distal end hood 231 of the endoscope 2 according to this embodiment, an elastic deformation portion 234 made of a thin portion thinner than the other portion of the hood main body 232 is formed at the distal end portion of the hood main body 232. The elastic deformation portion 234 is made from the same material as that for the hood main body 232. Further, such a configuration may be adopted that the elastic deformation portion 234 is formed with the U-shaped groove portions 215 shown in FIG. 7 or the openings 225 shown in FIG. 12 so that flexibility thereof is further raised. Incidentally, the portions other than the elastic deformation portion 234 in this embodiment are identical in configuration to those of the endoscope 2 according to the fourth embodiment, where the same portions as those in the endoscope 2 according to the fourth embodiment are attached with the same reference numerals and explanation thereof is omitted.

Therefore, the following effects can be achieved with the abovementioned configuration. That is, according to the embodiment, like the fourth embodiment, such an effect can be achieve even in this embodiment that the magnification (zoom) observation of a surface of a body tissue utilizing the second imaging unit 30, which is an ordinary observation optical system, can be performed stably even by the endoscope 2 provided with the first imaging unit 28, which is the observation optical system of an object contact type and which is brought into contact with a body tissue to observe the same, and object contact observation which brings the first imaging unit 28 of an object contact type in contact with a body tissue to observe the same can also be performed stably.

Further, according to the embodiment, since the elastic deformation portion 234 made of a thin portion thinner than the other portion of the hood main body 232 is formed at the distal end portion of the hood main body 232, the elastic deformation portion 234 can be made from the same material as that for the hood main body 232. Therefore, the number of assembly steps and the manufacturing cost can be reduced as compared with a case in which an elastic deformation portion separate from the hood main body 232 is assembled to the distal end portion of the hood main body 232.

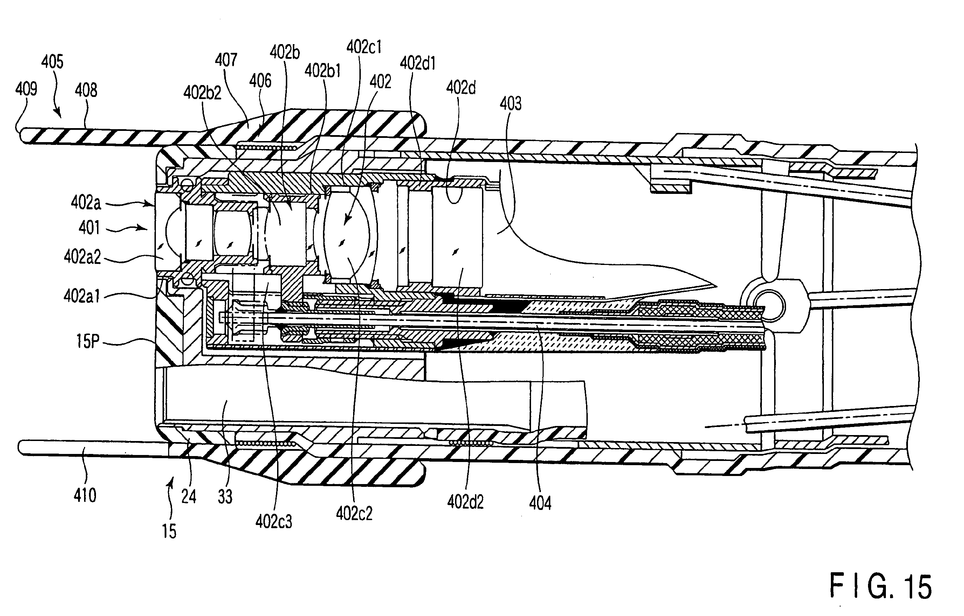

FIG. 15 shows a sixth embodiment of the present invention. This embodiment has such a configuration in which the endoscope 2 of the endoscope system 1 according to the first embodiment (see FIG. 1 to FIG. 6C) has been modified in the following manner. In FIG. 15, the same portions as those of the endoscope 2 according to the first embodiment are attached with the same reference numerals, and explanation thereof is omitted.

That is, an endoscope 2 according to this embodiment includes an imaging unit 401 of a single-lens type and of both a contact and separation type. The imaging unit 401 of both a contact and separation type has such a configuration in which a first observation portion of an object contact type (the first imaging unit 28 of an object contact type shown in the first embodiment) where observation is performed in a contact state with an analyte and a second observation portion (the second imaging unit 30 for ordinary observation) where observation is performed in a non-contact state with an analyte are integrated as an observation optical system.

The imaging unit 401 of both a contact and separation type includes a zoom lens unit 402 provided with a zoom optical system which can be switched between an object contact observation state having a monitor observation magnification range from about 200 to about 1000 and an ordinary observation state in which observation is performed in a non-contact state with an analyte, where an observation magnification can be changed continuously from a Tele (magnification) position to a Wide (wide-angle) position in the ordinary observation state, and an electric unit 403.

The zoom lens unit 402 further includes four (first to fourth) unit configuration bodies 402a to 402d. The first unit configuration body 402a includes a first lens frame 402a1 and a first lens group 402a2 configuring an objective lens.

The second unit configuration body 402b is a moving optical unit for zooming which is capable of advancing and retreating in an imaging optical axis direction. The second unit configuration body 402b includes a second lens frame (sliding lens frame) 402b1 and a second lens group (zoom lens) 402b2.

The third unit configuration body 402c includes a third lens frame 402c1 and a third lens group 402c2. A guide space 402c3 holding the second unit configuration body 402b so as to advance and retreat in the imaging optical axis direction is provided within the third lens frame 402c1 on the distal end side. The third lens group 402c2 is disposed rearward beyond the guide space 402c3. The fourth unit configuration body 402d includes a fourth lens 402d1 and a fourth lens group 402d2.

A distal end portion of a zoom wire 404 for operating the second unit configuration body 402b in the imaging optical axis direction in an advancing and retreating manner is fixed to the second lens frame 902b1 of the second unit configuration body 402b. An operation lever for zooming (not shown) provided on the operation portion of the endoscope is operated by a user so that the zoom wire 404 is driven in the imaging optical axis direction in an advancing and retreating manner. At this time, the second unit configuration body 402b, which is a zoom optical system, is moved forward (in the Wide (wide-angle) position direction) according to operation by which the zoom wire 404 is pushed out in the distal end direction. Further, the second unit configuration body 402b, which is a zoom optical system, is moved to the near side (in Tele (magnification) position direction) according to operation by which the zoom wire 404 is pulled in the near side direction. Here, a state in which the second unit configuration body 402b has been moved to a position other than the rearmost position of the guide space 402c3 of the third unit configuration body 402c is set in an observation range of the ordinary observation state where observation is performed in a non-contact state with the analyte. A state that the second unit configuration body 402b has been moved to the rearmost position of the guide space 402c3 is set in the object contact observation position (the object contact observation with the monitor observation magnification in a range from about 200 to 1000) where observation is performed in a contact state with the analyte.

Thereby, switching between the observation range of the ordinary observation state in which observation is performed in a non-contact state of the second unit configuration body 402b with an analyte, and the object contact observation position in which observation is performed in a contact state with an analyte, can be performed according to operation of the operation lever for zooming (not shown).

The projecting step portion 25 according to the first embodiment is not provided on the distal end portion 15 of the insertion portion 11 of the endoscope 2 according to this embodiment but a smooth flat surface 15p is formed over a whole distal end face of the distal end portion 15.

Further, a distal end hood 405 is attached on an outer peripheral surface of the distal end cover 24 at the distal end portion 15 of the insertion portion 11 of the endoscope 2 according to this embodiment. The distal end hood 405 includes a cylindrical hood main body 406. A mounting portion 407 mounted on an outer peripheral surface of the distal end cover 24 so as to be fitted thereon is provided at a proximal end portion of the hood main body 406. An elastically deformable elastic deformation portion 408 is disposed at the distal end portion of the hood main body 406.

The hood main body 406 has such a configuration that the mounting portion 407 mounted on an outer peripheral surface of a distal end portion 15 and the elastic deformation portion 408 are made from the same material. Further, the elastic deformation portion 408 has such a configuration that a thinner portion 409 is formed so as to be thinner regarding its wall portion than the mounting portion 407 mounted on the outer peripheral surface of the distal end portion 15 and a plurality of approximately U-shaped groove portions (notched portions) 410 notched in an approximate U shape are formed in the thin portion so that the flexibility of the wall portion is made larger than that of the mounting portion 407.

In the embodiment, at the time of ordinary endoscope observation, the zoom wire 404 is pushed out in the distal end direction and the second unit configuration body 402b, which is the zoom optical system of the imaging unit 401 of both the contact and separation type, is maintained in a state in which it has been moved forward (in the Wide (wide-angle) position direction). Ordinary endoscope observation utilizing the imaging unit 401 of both the contact and separation type is performed in the state that the second unit configuration body 402b has been held in the Wide (wide-angle) position.

The distal end portion of the distal end hood 405 is pushed onto an analyte H so as to surround a target site to be observed in a state that the distal end portion of the endoscope 2 has approached the target site to be observed. Thereby, an observation position at a magnification observation time utilizing the imaging unit 401 of both the contact and separation type is positioned. At this time, the second unit configuration body 402b, which is the zoom optical system, is moved toward the near side (in the Tele (magnification) position direction) according to operation by which the zoom wire 404 is pulled in the near side direction. Setting to a desired magnification is made. At the magnification observation time utilizing the imaging unit 401 of both the contact and separation type, the distal end portion of the elastic deformation portion 408 is disposed in the observation depth range A between the depth of field L1 on the far point side of the imaging unit 401 and the depth of field L2 on the near point side. In this state, the magnification observation utilizing the imaging unit 401 is performed.

At a contact observation time of a body tissue of an analyte H utilizing the imaging unit 401 of both the contact and separation type, a pressing force for pressing the distal end portion of the elastic deformation portion 408 of the distal end hood 405 to the analyte H is made further larger than that at the magnification observation time utilizing the imaging unit 401. Thereby, the elastic deformation portion 408 of the distal end hood 405 is shrunk and deformed in the striking direction. At this time, the elastic deformation portion 408 is shrunk and deformed in the axial direction to a state in which an outer peripheral surface side of the hood main body 406 is bent outwardly in an approximate L shape, so that the elastic deformation portion 408 is elastically deformed so as to be evacuated out of the field of view of the imaging unit 401 of both the contact and separation type. Thereby, the imaging unit 401 of both the contact and separation type and the illumination lens 91 of the first illumination window 29 are brought into contact with a surface of the body tissue of the analyte H such as a cell tissue to be observed. Thereby, object contact observation of high power where the distal end portion of the imaging unit 401 of both the contact and separation type is brought into contact with an object so that a cell tissue to be observed or the like is observed with high power, or the like is performed.

With the abovementioned configuration, the following effects can be achieved. That is, according to the embodiment, since the imaging unit 401 of a single-lens type and of both the contact and separation type is provided and the imaging unit 401 of both the contact and separation type provided with a zoom optical system which can be switched between an object contact observation state having a monitor observation magnification range from about 200 to about 1000 and an ordinary observation state in which observation is performed in a non-contact state with an analyte, in which the observation magnification can be continuously changed from the Tele (magnification) position to the Wide (wide-angle) position in the ordinary observation state is used, the entire installation space for the imaging unit can be reduced as compared with a case in which the imaging unit for object contact observation and the imaging unit for ordinary observation in which observation is performed in a non-contact state with an object are provided separately. Therefore, size reduction and diameter reduction of the distal end portion 15 of the endoscope 2 can be achieved.

Incidentally, the present invention is not limited to the abovementioned embodiments. For example, the elastic deformation portion 204 of the distal end hood 201 may be configured such that it is folded in a bellows manner. In each embodiment, the endoscope incorporated with the ordinary optical system using a solid-state imaging element such as a CCD or a CMOS as the imaging unit of super high power has been shown, but the present invention is also applicable to a confocal endoscope using a principle of a confocal microscope examination at a distal end portion of an endoscope instead of an endoscope incorporated into an ordinary optical system.

Next, other characteristic technical items not forming part of the invention will be additionally described below.

### (Additional Item 1)

A distal end hood for an endoscope including a projecting face projecting forward beyond an endoscope distal end face, comprising: a face holding a first contact state at an observation depth position on a near point side of ordinary observation; and a face holding a second contact state at the position of the projecting face.

### (Effect of Additional Item 1)

In a state in which an observation depth at a zoom time is shallow (about 0.5 mm), the first contact state can be held, while a stable contact state with a living body in the second contact state can be achieved at the time of living body contact observation. Accordingly, stable observation can be performed in both a zoom observation state and living body contact observation state.

### (Additional Item 2)

A distal end hood for an endoscope including a projecting face projecting forward beyond an endoscope distal end face, wherein the distal end hood has at least two kinds of resin hardness in a longitudinal direction of the distal end hood.

### (Effect of Additional Item 2)

Since contact of an end face of the distal end hood can be achieved in a state that the observation depth at a zoom time is shallow (about 0.5 mm), observation can be performed stably, and since the distal end hood is elastically deformed even at the time of living body contact observation, the endoscope distal end projecting portion can be easily brought into contact with a living body. Accordingly, stable observation can be performed in both a zoom observation state and living body contact observation state.

### (Additional Item 3)

The distal end hood according to Additional Item 2, wherein a distal end portion of the distal end hood is softer in resin hardness than a proximal end portion of the distal end hood.

### (Additional Item 4)

A hood for an endoscope comprising a mounting portion mounted on an endoscope distal end portion and a striking face provided at a leading face, and a deformable portion which is shrunk and deformed in a striking direction by further exerting a striking force from a state in which the striking face has been struck on a striking-target object.

### (Additional Item 5)

The hood for an endoscope according to Additional Item 4, wherein a second striking face provided rearward behind a first striking face is struck on the striking-target object according to shrinking and deformation of the deformable portion.

### (Additional Item 6)

The hood for an endoscope according to Additional Item 4 or 5, wherein the deformable portion is shrunk and deformed such that the first striking face is evacuated outwardly.

### (Additional Item 7)

An endoscope with a hood comprising: a distal end hard portion including a first observation optical system and a second observation optical system provided at a position projecting forward beyond the first observation optical system on a distal end face thereof; and a distal end hood which is provided so as to extend from the distal end hard portion forward beyond the second observation optical system and can be shrunk and deformed up to the vicinity of the second observation optical system according to rearward biasing force.

### Industrial Applicability

The present invention is effective, for example, in a technical field in which an endoscope is provided with an observation optical system for ordinary observation and an observation optical system of an object contact type where a distal end portion of an objective optical system is brought into contact with an object to observe the object, where the endoscope is inserted into a body cavity for observation, and the technical field of endoscope manufacturing.

## Claims

1. An endoscope (2) with a hood comprising:
an insertion portion (11) inserted into a lumen;
a projecting face (25a) provided at a distal end portion (15) of the insertion portion (11) so as to project;
a first observation portion (28) disposed on the projecting face (25a) and performing observation in a contact state of an observation optical system with an analyte (H);
a second observation portion (30) disposed rearward beyond the projecting face (25a), and performing observation in a non-contact state of the observation optical system with the analyte (H) and allowing switching between ordinary observation state for wide-angle observation and a magnification observation state for magnification observation; and
a distal end hood (201) including a cylindrical hood main body (202) whose proximal end portion is attached on an outer peripheral surface of the distal end portion (15) and an elastic deformation portion (204) provided at a distal end portion of the hood main body (202) so as to extend forward beyond the projecting face (25a) and which is elastically deformable; wherein
the elastic deformation portion (204) is configured such that a distal end portion of the elastic deformation portion (204) abuts on the analyte (H) to position an observation position of the second observation portion (30) at the magnification observation time utilizing the second observation portion (30) while the elastic deformation portion (204) is elastically deformable up to a position where the first observation portion (208) abuts on the analyte (H) at a contact observation time utilizing the first observation portion (28).

2. The endoscope (2) with a hood according to claim 1, wherein
the hood main body (202) has such a configuration in which a proximal end portion attached on an outer peripheral surface of the distal end portion (15) is formed integrally with a distal end cover (24) covering the outer peripheral surface of the distal end portion (15).

3. The endoscope (2) with a hood according to claim 1, wherein
the hood main body (202) is configured such that the distal end portion of the elastic deformation portion (204) is disposed in an observation depth range between a field of view on a far point side of the second observation portion (30) and a field of view on a near point side at the magnification observation time utilizing the second observation portion (30).

4. The endoscope (2) with a hood according to claim 1, wherein
the hood main body (202) has such a configuration in which a mounting portion (203) mounted on the outer peripheral surface of the distal end portion (15) and the elastic deformation portion (204) are formed as separate parts, respectively.

5. The endoscope (2) with a hood according to claim 4, wherein
the elastic deformation portion (204) is made from a material softer than that for the mounting portion (203).

6. The endoscope (2) with a hood according to claim 1, wherein
the hood main body (202) has such a configuration in which a mounting portion (203) mounted on the outer peripheral surface of the distal end portion (15) and the elastic deformation portion (204) are made from the same material, and
the elastic deformation portion (204) is made of a thin portion smaller in thickness of a wall portion than the mounting portion (203).

7. The endoscope (2) with a hood according to claim 1, wherein
the elastic deformation portion (204) is configured such that a position of a proximal end portion thereof is disposed rearward beyond a distal end position of the first observation portion (28).

8. An endoscope (2) with a hood comprising:
an insertion portion (11) inserted into a lumen;
a zoom optical system which is disposed at a distal end portion of the insertion portion (11) and can be switched between a contact observation state in which observation is performed in a contact state of an observation optical system with an analyte (H) and a non-contact observation state in which observation is performed in a non-contact state of the observation optical system with the analyte (H), wherein the observation optical system can be switched between an ordinary observation state for wide-angle observation and a magnification observation state for magnification observation at the non-contact observation state time; and
a distal end hood (201) including a cylindrical hood main body (202) whose proximal end portion is attached on an outer peripheral surface of the distal end portion (15) and an elastic deformation portion (204) provided at a distal end portion of the hood main body (202) so as to extend forward beyond a projecting face (25a) and which is elastically deformable, wherein
the elastic deformation portion (204) is configured such that a distal end portion of the elastic deformation portion (204) abuts on the analyte (H) to position an observation position at the magnification observation time of the zoom optical system at the magnification observation time, while the elastic deformation portion (204) is elastically deformable up to a position where the zoom optical system abuts on the analyte (H) at the contact observation time of the zoom optical system.

## Patentansprüche

1. Endoskop (2) mit einer Haube, die aufweist:
einen Einführabschnitt (11), der in ein Lumen eingeführt wird;
eine vorstehende Anlagefläche (25a), die bei einem Distalendabschnitt (15) des Einführungsabschnitts (11) so angeordnet ist, dass diese abragt;
einen ersten Beobachtungsabschnitt (28), der auf der abragenden Anlagefläche (25a) angeordnet ist und einen Beobachtungsvorgang ausführt in einem Kontaktzustand eines Beobachtungsoptiksystems mit einem Analyt (H);
einem zweiten Beobachtungsabschnitt (30), der rückwärtig hinter der vorstehenden Anlagefläche (25a) angeordnet ist und einem Beobachtungsvorgang ausführt in einem Nichtkontaktierungszustand des Beobachtungsoptiksystems mit dem Analyt (H), und der zwischen einem gewöhnlichen Beobachtungszustand für Weitwinkelbeobachtung und einen Vergrößerungs-Beobachtungszustand für Vergrößerungsbeobachtung hin- und herschaltbar ist; und
einer Distalendhaube (201), die einen zylindrischen Haubenhauptkörper (202) aufweist, dessen Proximalendabschnitt auf einer äußeren Begrenzungsoberfläche des Distalendabschnitts (15) angebracht ist, und einen elastisch deformierbaren Abschnitt (204) aufweist, der an einem Distalendabschnitt des Haubenhauptkörpers (202) vorgesehen ist, derart, dass dieser nach vorne über die vorstehende Anlagefläche (25a) hinausragt; wobei
der elastisch deformierbare Abschnitt (204) so ausgestaltet ist, dass ein Distalendabschnitt des elastisch deformierbaren Abschnitts (204) auf den Analyt (H) stößt, um eine Beobachtungsposition des zweiten Beobachtungsabschnitts (30) zur Vergrößerungsbeobachtungszeit zu positionieren unter Verwendung des zweiten Beobachtungsabschnitts (30), wobei der elastisch deformierbare Abschnitt (204) bis zu einer Position elastisch deformierbar ist, bei der der erste Beobachtungsabschnitt (28) auf den Analyt (H) stößt zu einer Kontaktbeobachtungszeit unter Verwendung des ersten Beobachtungsabschnitts (28).

2. Endoskop mit einer Haube nach Anspruch 1, wobei
der Haubenhauptkörper (202) so ausgestaltet ist, dass ein Proximalendabschnitt, der auf einer äußeren Begrenzungsoberfläche des DistalEndabschnitts (15) angebracht ist, zusammen mit einer Distalendabdeckung (24), die die äußere Begrenzungsoberfläche des Distalendabschnitts (15) bedeckt, einstückig ausgebildet ist.

3. Endoskop mit einer Haube nach Anspruch 1, wobei
der Haubenhauptkörper (202) so ausgebildet ist, dass der Distalendabschnitt des elastisch deformierbaren Abschnitts (204) angeordnet ist in einem Beobachtungstiefenbereich zwischen einem Sichtfeld auf einer Fernpunktseite des zweiten Beobachtungsabschnitts (30) und einem Sichtfeld auf einer Nahpunktseite zur Vergrößerungsbeobachtungszeit unter Verwendung des zweiten Beobachtungsabschnitts (30).

4. Endoskop mit einer Haube nach Anspruch 1, wobei
der Haubenhauptkörper (202) so ausgestaltet ist, dass ein Montierabschnitt (203), der auf der äußeren Begrenzungsoberfläche des Distalendabschnitts (15) angebracht ist, und der elastisch deformierbare Abschnitt (204) als separate Teile ausgebildet sind.

5. Endoskop (2) mit einer Haube nach Anspruch 4, wobei
der elastisch deformierbare Abschnitt (204) aus einem Material hergestellt ist, das weicher ist als das des Montierabschnitts (203).

6. Endoskop (2) mit einer Haube gemäß Anspruch 1, wobei der Haubenhauptkörper (202) so ausgestaltet ist, dass ein Montierabschnitt (203), der auf die äußere Begrenzungsoberfläche des Distalendabschnitts (15) aufgebracht ist, und der elastisch deformierbare Abschnitt (204) aus demselben Material gefertigt sind, und wobei
der elastisch deformierbare Abschnitt (204) aus einem dünnen Abschnitt gefertigt ist, der eine geringere Dicke eines Wandabschnitts aufweist als der Montierabschnitt (203).

7. Endoskop (2) mit einer Haube nach Anspruch 1, wobei
der elastisch deformierbare Abschnitt (204) so ausgestattet ist, dass eine Position eines entsprechenden Proximalendabschnitts rückseitig hinter einem Distalendabschnitt des ersten Beobachtungsabschnitts (28) angeordnet ist.

8. Endoskop (2) mit einer Haube, aufweisend:
einen Einführungsabschnitt (11), der in ein Lumen eingeführt wird,
ein Zoomoptiksystem, das bei einem Distalendabschnitt des Einführungsabschnitts (11) angeordnet ist und hin- und herschaltbar ist zwischen einem Kontaktbeobachtungszustand, in dem Beobachtung ausgeführt wird während eines Kontaktzustands eines Beobachtungsoptiksystems mit einem Analyt (H), und einem Nichtkontaktierungsbeobachtungszustand, in dem Beobachtung ausgeführt wird in einem Nichtkontaktierungszustand des Beobachtungsoptiksystems mit dem Analyt (H), wobei das Beobachtungsoptiksystem hin- und herschaltbar ist zwischen einem gewöhnlichen Beobachtungszustand für Weitwinkelbeobachtung, und einem Vergrößerungsbeobachtungszustand für Vergrößerungsbeobachtung zur Nichtkontaktbeobachtungszustandszeit; und
einer Distalendehaube (201), aufweisend einen zylindrischen Haubenhauptkörper (202), dessen Proximalendabschnitt mit einer äußeren Begrenzungsoberfläche des Distalendabschnitts (15) verbunden ist, und einem elastisch deformierbaren Abschnitt (204), der an einem Distalendabschnitt des Haubenhauptkörpers (202) so angeordnet ist, dass sich dieser vorwärts über eine vorstehende Anlagefläche (25a) hinaus erstreckt;
wobei der elastisch deformierbare Abschnitt (204) so ausgebildet ist, dass ein Distalendabschnitt des elastisch deformierbaren Abschnitts (204) auf den Analyt (H) stößt, um einen Beobachtungsabschnitt des Zoomoptiksystems zur Vergrößerungsbeobachtungszeit zu positionieren, während der elastisch deformierbare Abschnitt (204) elastisch deformierbar ist bis zu einer Position, in der das Zoomoptiksystem auf den Analyt (H) stößt zur Kontaktbeobachtungszeit des Zoomoptiksystems.

## Revendications

1. Endoscope (2) avec un capuchon comprenant:
une partie d'insertion (11) insérée dans une lumière;
une face de projection (25a) prévue au niveau d'une partie d'extrémité distale (15) de la partie d'insertion (11) de façon à faire saillie;
une première partie d'observation (28) disposée sur la face de projection (25a) et réalisant l'observation dans un état de contact d'un système d'observation optique avec une substance à analyser (H);
une deuxième partie d'observation (30) disposée vers l'arrière au-delà de la face de projection (25a), et réalisant une observation dans un état de non contact du système d'observation optique avec la substance à analyser (H) et permettant la commutation entre un état d'observation ordinaire pour une observation grand angulaire et un état d'observation de grossissement pour une observation de grossissement; et
un capuchon d'extrémité distale (201) comprenant un corps principal de capuchon cylindrique (202) dont la partie d'extrémité proximale est fixée sur une surface périphérique externe de la partie d'extrémité distale (15) et une partie de déformation élastique (204) prévue au niveau d'une partie d'extrémité distale du corps principal de capuchon (202) de façon à s'étendre vers l'avant au-delà de la face de projection (25a) et qui est électriquement déformable; dans lequel
la partie de déformation élastique (204) est configurée d'une manière telle qu'une partie d'extrémité distale de la partie de déformation élastique (204) vient en butée contre la substance à analyser (H) pour positionner une position d'observation de la deuxième partie d'observation (30) au moment de l'observation de grossissement en utilisant la deuxième partie d'observation (30) alors que la partie de déformation élastique (204) est élastiquement déformable jusqu'à une position dans laquelle la première partie d'observation (28) vient en butée contre la substance à analyser (H) à un moment d'observation de contact en utilisant la première partie d'observation (28).

2. Endoscope (2) avec un capuchon selon la revendication 1, dans lequel
le corps principal de capuchon (202) présente une configuration dans laquelle une partie d'extrémité proximale fixée sur une surface périphérique externe de la partie d'extrémité distale (15) est formée solidairement avec un couvercle d'extrémité distale (24) recouvrant la surface périphérique externe de la partie d'extrémité distale (15).

3. Endoscope (2) avec un capuchon selon la revendication 1, dans lequel
le corps principal de capuchon (202) est configuré d'une manière telle que la partie d'extrémité distale de la partie de déformation élastique (204) est disposée dans une plage de profondeur d'observation située entre un champ de vue du côté d'un point éloigné de la deuxième partie d'observation (30) et un champ de vue du côté d'un point proche au moment de l'observation de grossissement utilisant la deuxième partie d'observation (30).

4. Endoscope (2) avec un capuchon selon la revendication 1, dans lequel
le corps principal de capuchon (202) présente une configuration dans laquelle une partie de montage (203) montée sur la surface périphérique externe de la partie d'extrémité distale (15) et la partie de déformation élastique (204) sont formées en tant que parties séparées, respectivement.

5. Endoscope (2) avec un capuchon selon la revendication 4, dans lequel
la partie de déformation élastique (204) est constituée à partir d'un matériau plus mou que celui pour la partie de montage (203).

6. Endoscope (2) avec un capuchon selon la revendication 1, dans lequel
le corps principal de capuchon (202) présente une configuration dans laquelle une partie de montage (203) montée sur la surface périphérique externe de la partie d'extrémité distale (15) et la partie de déformation élastique (204) sont constitués à partir du même matériau, et
la partie de déformation élastique (204) est constituée d'une partie fine dont la partie de paroi présente une épaisseur plus faible que la partie de montage (203).

7. Endoscope (2) avec un capuchon selon la revendication 1, dans lequel
la partie de déformation élastique (204) est configurée d'une manière telle qu'une position de sa partie d'extrémité proximale est disposée vers l'arrière au-delà d'une position d'extrémité distale de la première partie d'observation (28).

8. Endoscope (2) avec un capuchon comprenant:
une partie d'insertion (11) insérée dans une lumière;
un système de zoom optique qui est disposé au niveau d'une partie d'extrémité distale de la partie d'insertion (11) et qui peut être commuté entre un état d'observation de contact dans lequel l'observation est exécutée dans un état de contact d'un système d'observation optique avec une substance à analyser (H) et un état d'observation de non contact dans lequel l'observation est exécutée dans un état de non contact du système d'observation optique avec la substance à analyser (H), dans lequel le système d'observation optique peut être commuté entre un état d'observation ordinaire avec une observation grand angulaire et un état d'observation de grossissement pour une observation de grossissement au moment de l'état d'observation de non contact; et
un capuchon d'extrémité distale (201) incluant un corps principal de capuchon cylindrique (202) dont la partie d'extrémité proximale est fixée sur une surface périphérique externe de la partie d'extrémité distale (15) et une partie de déformation élastique (204) prévue au niveau d'une partie d'extrémité distale du corps principal de capuchon (202) de façon à s'étendre vers l'avant au-delà d'une face de projection (25a) et qui est élastiquement déformable, dans lequel
la partie de déformation élastique (204) est configurée d'une manière telle qu'une partie d'extrémité distale de la partie de déformation élastique (204) vient en butée contre la substance à analyser (H) pour positionner une position d'observation au moment de l'observation de grossissement du système de zoom optique au moment de l'observation de grossissement, alors que la partie de déformation élastique (204) est élastiquement déformable jusqu'à une position dans laquelle le système de zoom optique vient en butée contre la substance à analyser (H) au moment de l'observation de contact du système de zoom optique.
